(19) Europäisches Patentamt
European Patent Office
Office européen des brevets

(11) Publication number : **0 554 025 A2**

# (12) EUROPEAN PATENT APPLICATION

(21) Application number : **93300496.2**

(22) Date of filing : **25.01.93**

(51) Int. Cl.[5] : **C07D 473/00,** C07C 43/12, C07C 43/13, C07D 303/08

(30) Priority : **27.01.92 US 826585**

(43) Date of publication of application : **04.08.93 Bulletin 93/31**

(84) Designated Contracting States : **AT BE CH DE DK ES FR GB GR IE IT LI LU MC NL PT SE**

(71) Applicant : **E.R. SQUIBB & SONS, INC.
Lawrenceville-Princeton Road
Princeton New Jersey 08543-4000 (US)**

(72) Inventor : **Vite, Gregory D.
8 Blackfoot Road
Trenton, NJ (US)**
Inventor : **Zahler, Robert
5 E. Welling Avenue
Pennington, NJ (US)**
Inventor : **Slusarchyk, William A.
19 Richmond Drive
Skillman, NJ (US)**
Inventor : **Tino, Joseph A.
1 Devon Ct.
Robbinsville, NJ (US)**
Inventor : **Singh, Janak
F13 Shirley Lane
Lawrenceville, NJ (US)**
Inventor : **Kissick, Thomas P.
10 Sherman Pl.
Lawrenceville, NJ (US)**

(74) Representative : **Thomas, Roger Tamlyn et al
D. Young & Co. 10 Staple Inn
London WC1V 7RD (GB)**

## (54) Fluorinated cyclobutyl purines and pyrimidines.

(57) Antiviral activity is exhibited by compounds having the formula

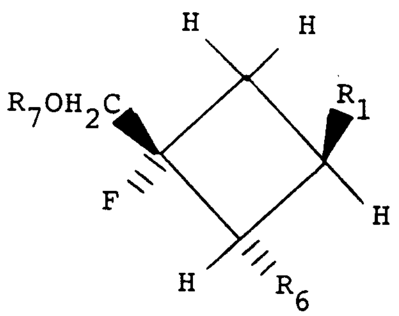

or a pharmaceutically acceptable salt thereof wherein $R_1$ is a purinyl, substituted purinyl, pyrimidinyl, or substituted pyrimidinyl, $R_6$ is -OH, $-CH_2OH$, $-OPO_3H_2$, $-CH_2-O-PO_3H_2$,

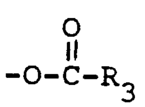,

or

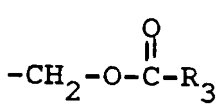,

and $R_7$ is hydrogen, $-PO_3H_2$, or

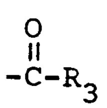.

EP 0 554 025 A2

Zahler et al. in U.S. Patents 4,855,466 and 4,918,075 disclose antiviral purinyl and pyrimidinyl cyclobutanes having a hydroxy and a hydroxymethyl substituent.

Slusarchyk et al. in European Patent Application No. 335,355 and U.S. Patents 5,126,345 and 5,130,462, Ichikawa et al. in Europe an Patent Application No. 358,154, Norbeck et al. in European Patent Application No. 366,059, and Bisacchi et al. in U.S. Patent 5,064,961 disclose antiviral purinyl and pyrimidinyl bis(hydroxymethyl)cyclobutanes.

Zahler et al. in European Patent Application 458,363 disclose antiviral 2-fluoro-3,4-bis(hydroxymethyl)cyclobutanes having a purinyl or pyrimidinyl substituent at the 1-position.

This invention is directed to antiviral 3-fluoro-3-hydroxymethyl-2-(hydroxy or hydroxymethyl)cyclobutanes having a purinyl or pyrimidinyl substituent at the 1-position, intermediates, and processes for making such compounds.

The antiviral compounds of this formula are of the formula

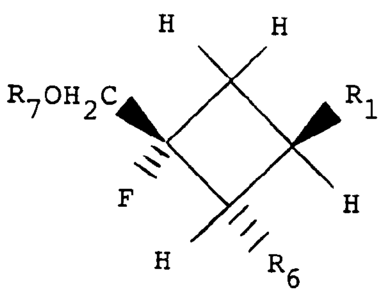

1

or a pharmaceutically acceptable salt thereof. In formula 1, and throughout the specification, the symbols are as defined below.

$R_1$ is

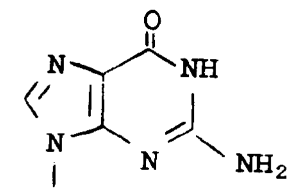

,

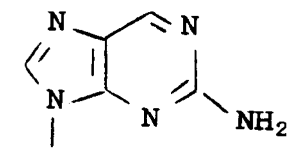

,

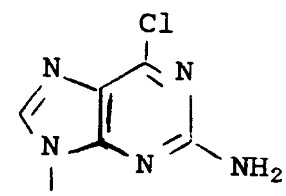

,

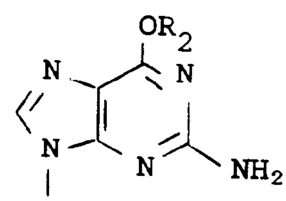

,

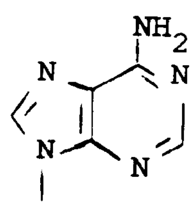

,

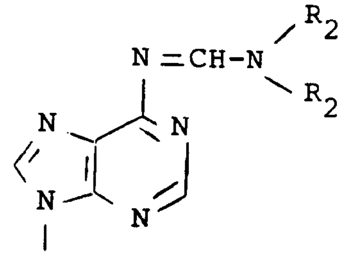

,

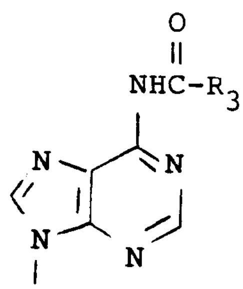

,

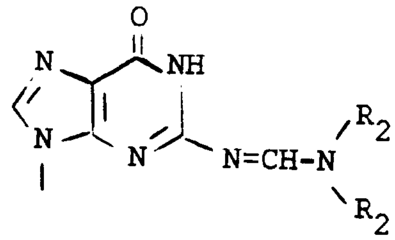 , 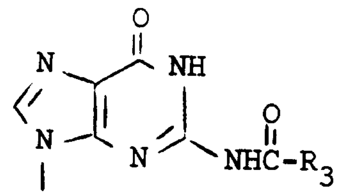 ,

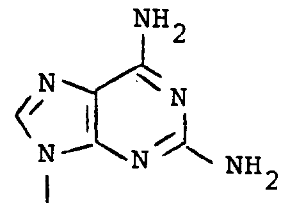 , 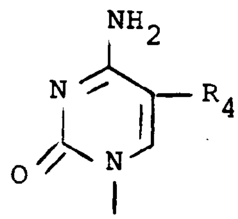 , or

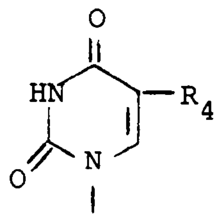 .

$R_2$ is alkyl.

$R_3$ is hydrogen, alkyl, substituted alkyl, or aryl.

$R_4$ is fluoro, chloro, bromo, iodo, hydrogen, methyl, trifluoromethyl, ethyl, n-propyl, 2-fluoroethyl, 2-chloroethyl, or

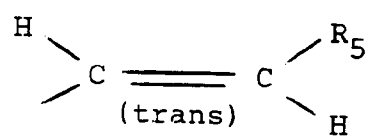


wherein $R_5$ is chloro, bromo, iodo, hydrogen, methyl, or trifluoromethyl.

$R_6$ is -OH, $-CH_2OH$, $-OPO_3H_2$, $-CH_2-OPO_3H_2$,

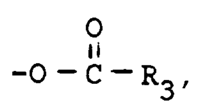 or 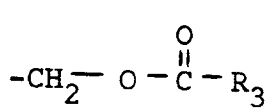 .

$R_7$ is hydrogen, $-PO_3H_2$, or

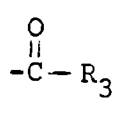 .

The term "alkyl" refers to straight and branched chain groups of 1 to 10 carbons. The term "substituted alkyl" refers to such alkyl groups of 1 to 10 carbons having one or more, preferably one, two or three substituents selected from bromo. chloro. fluoro, iodo, amino, hydroxy, cyano, trialkylamino wherein each alkyl is of 1 to 6 carbons, alkoxy of 1 to 6 carbons, aryl, and carboxy. The term "aryl" refers to phenyl and phenyl having one, two, or three substituents, preferably one, selected from alkyl of 1 to 6 carbons, alkoxy of 1 to 6 carbons, bromo, chloro, fluoro, iodo, trifluoromethyl, amino, alkylamino of 1 to 6 carbons, dialkylamino wherein each alkyl is of 1 to 6 carbons, nitro, cyano, alkanoyloxy of 2 to 11 carbons, carboxy, carbamoyl, and hydroxy.

The compounds of formula 1, and the pharmaceutically acceptable salts thereof, are antiviral agents that can be used to treat viral infection in mammalian species such as domesticated animals (e.g., dogs, cats, horses and the like) and humans, and avian species (e.g., chickens and turkeys). The compounds of formula 1 wherein $R_1$ is

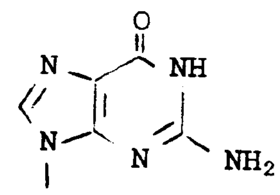

are effective against one or more of the following viruses: herpes simplex virus 1 and 2, varicellazoster virus and cytomegalovirus. They are also believed to be active against a variety of other DNA viruses. Exemplary DNA viruses in addition to those named above include other herpes viruses (e.g., Epstein-Barr virus, pseudorabies virus, human herpes virus 6, and the like), poxviruses (e.g., vaccinia virus, monkey pox and myoma), papovaviruses (e.g., the papilloma viruses), hepatitis B virus, and adenoviruses.

The other compounds of formula 1 are believed to be active against one or more of the following viruses: herpes simplex virus 1 and 2, varicella-zoster virus, cytomegalovirus, human immuno deficiency viruses (HIV), and the other DNA viruses described above.

The compounds of this invention may be administered parenterally (for example, by intravenous, intraperitoneal or intramuscular injection), orally or topically.

The compounds may be administered orally or parenterally in an amount effective to treat the infection. The dosage will, of course, depend on the severity of the infection, but will likely be in the range of about 1.0 to 50 mg/kg of body weight. The desired dose may be administered several times daily at appropriate intervals.

For infections of the eye, or other external tissues (e.g., mouth and skin) the compositions may be applied to the infected part of the body of the patient topically as an ointment, cream, aerosol, gel, powder, lotion, suspension or solution (e.g., as in eye drops). The concentration of the compound in the vehicle will, of course, depend on the severity of the infection, but will likely be in the range of about 0.1 to 7% by weight.

A compound of formula 1 wherein $R_1$ is

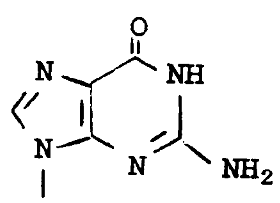

$R_6$ is $-CH_2OH$ and $R_7$ is hydrogen can be prepared from an intermediate of formula

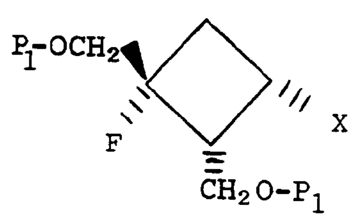

2

wherein $P_1$ is a protecting group such as benzyl or silyl, and X is a leaving group such as a straight or branched chain lower alkyl of 1 to 5 carbons, phenyl, substituted lower alkyl or substituted phenyl sulfonate well known in the art (e.g., benzenesulfonyloxy, p-toluenesulfonyloxy, methanesulfonyloxy, p-nitrophenylsulfonyloxy, or trifluoromethylsulfonyloxy). The term "silyl" refers to silyl protecting groups well known in the art [e.g., t-butyldimethylsilyl, t-butyldiphenylsilyl, (triphenylmethyl)dimethylsilyl, methyldiisopropylsilyl, or triisopropylsilyl].

Reaction of a compound of formula 2 with a protected form of guanine such as a compound of formula

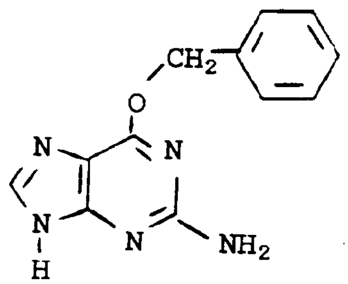

3

in the presence of a base such as potassium carbonate, sodium hydride, lithium hydride, or potassium hydride in an aprotic polar solvent such as dimethylformamide, dimethylsulfoxide, or sulfolane (tetramethylene sulfone) yields the corresponding compound of formula

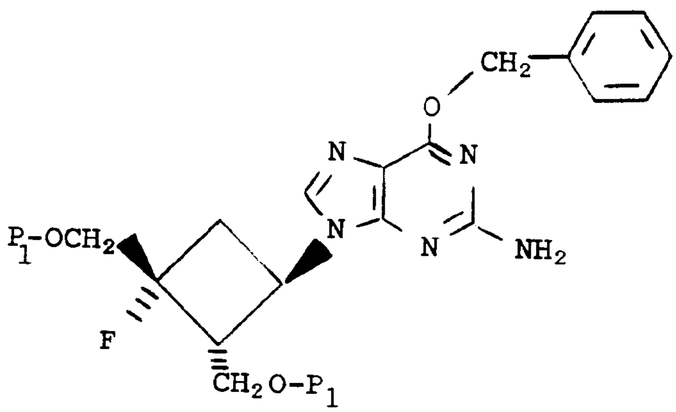

4

Optionally, the reaction can be run in the presence of a metal chelating catalyst such as 18-crown-6 (1,4,7,10,13,16-hexaoxacyclooctadecane) or 15-crown-5 (1,4,7,10,13-pentaoxacyclopentadecane).

Alternatively, treatment of a compound of formula 2 with a tetraalkylammonium salt of a compound of formula 3 (in an aprotic solvent such as methylene chloride, acetonitrile, dimethylformamide, dimethylsulfoxide or sulfolane) yields a compound of formula 4. A tetraalkylammonium salt of a compound of formula 3 can be prepared by treatment of a compound of formula 3 (in an aprotic, polar solvent such as dimethylformamide) with a tetraalkylammonium hydroxide such as aqueous tetra-n-butylammonium hydroxide and subsequent azeotropic removal of water. Alternatively, a tetraalkylammonium salt of a compound of formula 3 can be prepared by treatment of a compound of formula 3 in a chlorinated hydrocarbon solvent such as methylene chloride with a tetraalkylammonium hydroxide (such as aqueous tetra-n-butylammonium hydroxide). Removal of the water layer, drying of the organic layer (with a drying agent such as sodium or magnesium sulfate), filtration, and subsequent removal of the volatiles under vacuum provides a tetraalkylammonium salt of a compound of formula 3.

Removal of the protecting groups from a compound of formula 4 yields a compound of formula 1 wherein $R_1$ is

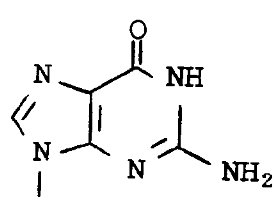

$R_6$ is $-CH_2OH$ and $R_7$ is hydrogen.

When the group P in compound 4 is a silyl protecting group, removal of the $P_1$ group can be accomplished using fluoride ion (e.g., tetrabutylammonium fluoride in tetrahydrofuran). The purine O-benzyl protecting group can then be removed with aqueous alcoholic mineral acid or by hydrogenolysis (e.g., palladium hydroxide on carbon in cyclohexene and ethanol). When the protecting group $P_1$ in 4 is benzyl, removal of all the benzyl protecting groups can be effected using sodium in liquid ammonia, hydrogenolysis (e.g., palladium hydroxide on carbon in cyclohexene and ethanol), or boron trichloride in dichloromethane.

A compound of formula 1 wherein

$R_1$ is

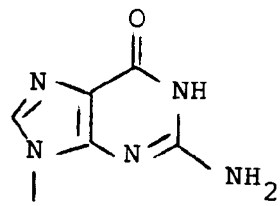

$R_6$ is -OH and $R_7$ is hydrogen can be prepared by reaction of an intermediate of the formula

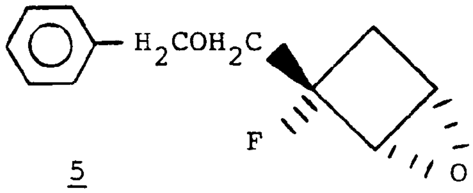

5

with a compound of formula 3 in the presence of a base such as potassium carbonate, sodium hydride, lithium hydride, or potassium hydride in an aprotic, polar solvent such as dimethylformamide, dimethylsulfoxide or sulfolane yielding the compound of the formula

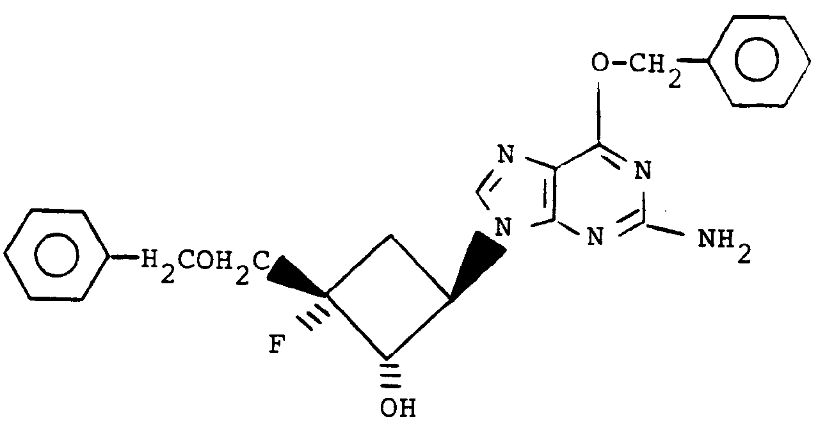

.

6

Alternatively, treatment of the compound of formula 5 with a tetraalkylammonium salt of the compound of formula 3 (in an aprotic solvent such as methylene chloride, acetonitrile, dimethylformamide, dimethylsulfoxide, or sulfone) yields the compound of formula 6.

Removal of the benzyl protecting groups from the compound of formula 6 [using sodium in liquid ammonia, hydrogenolysis (e.g., palladium hydroxide on carbon in cyclohexane and ethanol), or boron trichloride in dichloromethane] provides a compound of formula 1 wherein $R_1$ is

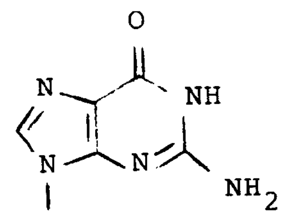

$R_6$ is -OH and $R_7$ is hydrogen.

Reaction of a compound of formula 2 with the compound

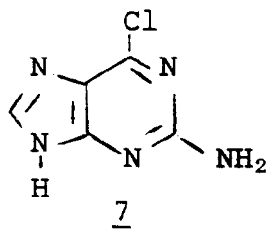

7

under conditions analogous to those used in the preparation of compound 4 provides a compound of formula

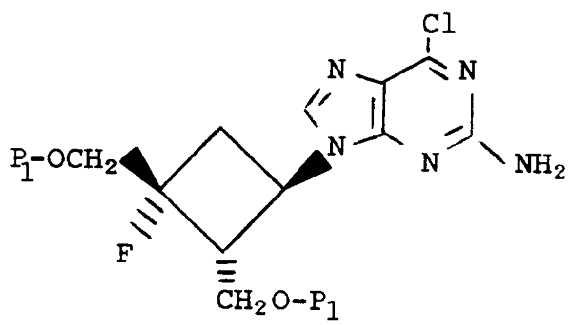

.

8

Removal of the protecting groups $P_1$ provides a compound of formula 1 wherein $R_1$ is

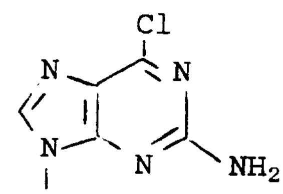

$R_6$ is $-CH_2OH$ and $R_7$ is hydrogen. For example, when the protecting group $P_1$ in 8 is silyl, the protecting group can be removed by treatment with fluoride ion (e.g., tetrabutylammonium fluoride). When the protecting group $P_1$ in 8 is benzyl, removal of the $P_1$ group can be performed by treatment with boron trichloride.

Acid hydrolysis (e.g., using hot aqueous hydrochloric acid) of the chloro group of a compound of formula 1 or methanolysis (e.g., using sodium methoxide in methanol) of the chloro group of a compound of formula 1 followed by acid hydrolysis wherein $R_1$ is

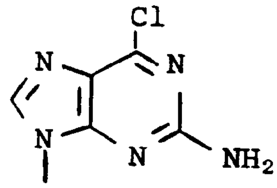

$R_6$ is $-CH_2OH$ and $R_7$ is hydrogen provides a compound of formula 1 wherein $R_1$ is

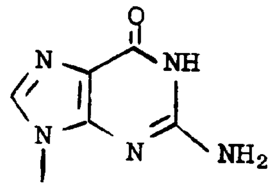

$R_6$ is $-CH_2OH$ and $R_7$ is hydrogen.

Reaction of the compound of formula 5 with a compound of formula 7 under conditions analogous to those used in the preparation of a compound of formula 6 provides the compound of formula

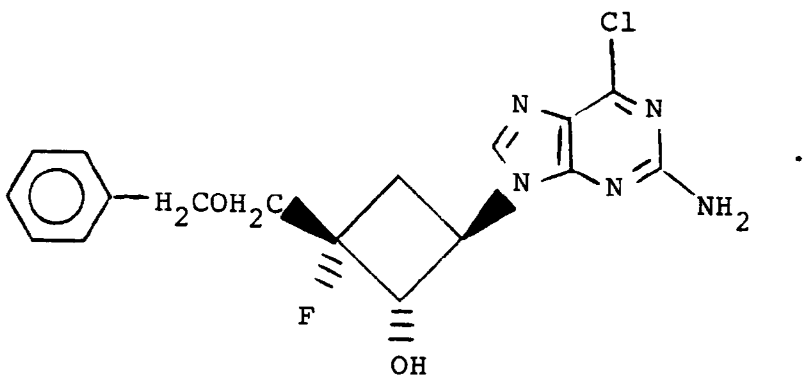

9

Removal of the benzyl protecting group from the compound of formula 9 with boron trichloride provides a compound of formula 1 wherein $R_1$ is

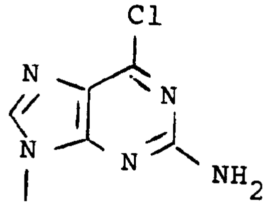

$R_6$ is -OH and $R_7$ is hydrogen.

Acid hydrolysis (e.g., using hot aqueous hydrochloric acid) of the chloro group of a compound of formula 1 wherein $R_1$ is

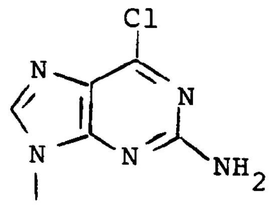

$R_6$ is -OH and $R_7$ is hydrogen, or methanolysis (e.g., using sodium methoxide in methanol) of the chloro group in this compound of formula 1 followed by acid hydrolysis provides a compound of formula 1 wherein $R_1$ is

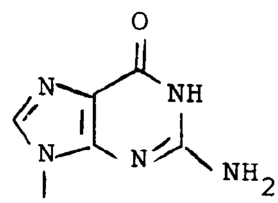

$R_6$ is -OH and $R_7$ is hydrogen.

A compound of formula 1 wherein $R_1$ is

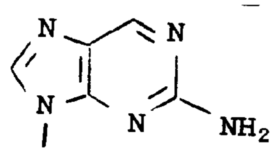

$R_6$ is $-CH_2OH$ and $R_7$ is hydrogen can be prepared from a compound of formula 8. For example, when the $P_1$ group in 8 is a silyl protecting group, the chloro group can first be reduced by hydrogenation (e.g., ammonium formate and palladium on carbon in methanol or ethanol, palladium on carbon in cyclohexene and ethanol, or palladium on carbon, hydrogen and ethanol), and then the protecting group $P_1$ can be removed using fluoride ion. Alternatively, the silyl protecting groups $P_1$ can be removed first and then the chloro group can be reduced. When the protecting group $P_1$ in 8 is benzyl, deprotection and reduction of the chloro group can be accomplished simultaneously by hydrogenolysis (e.g., palladium hydroxide on carbon in cyclohexene and ethanol; or ammonium formate or formic acid and palladium on carbon in methanol or ethanol).

Alternatively, this compound of formula 1 can be prepared by reacting an optionally protected compound of formula

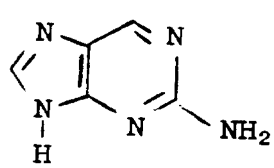

10

with a compound of formula 2 according to procedures analogous to those used in the preparation of a compound of formula 4, followed by removal of the protecting groups by methods known in the art. An optionally protected form of compound 10 can be protected at the amino ($-NH_2$) group by such exemplary groups as acyl (e.g., acetyl or benzoyl), trityl, or substituted trityl (e.g., 4-monomethoxytrityl and 4,4'-dimethoxytrityl).

A compound of formula 1 wherein $R_1$ is

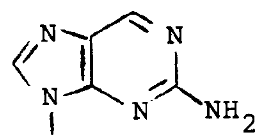

$R_6$ is -OH and $R_7$ is hydrogen can be prepared from a compound of formula 9. Reduction of the chloro group and the simultaneous removal of the benzyl protecting group in 9 can be accomplished by hydrogenolysis (e.g., palladium hydroxide on carbon in cyclohexane and ethanol).

Alternatively, this compound of formula 1 can be prepared by reacting an optionally protected compound of formula 10 with a compound of formula 5 according to procedures analogous to those used in the preparation of a compound of formula 6 followed by removal of the benzyl protecting group by methods known in the art. An optionally protected form of compound 10 can be protected at the amino ($-NH_2$) group by such exemplary groups as acyl, trityl, or substituted trityl such as 4-monomethoxytrityl and 4,4'-dimethoxytrityl.

A compound of formula 1 wherein $R_1$ is

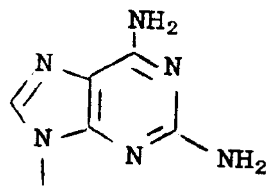

$R_6$ is $-CH_2OH$ and $R_7$ is hydrogen can be prepared from a compound of formula 8 by methods known in the art, for example,treatment with hot methanolic ammonia followed by removal of the protecting groups $P_1$ (e.g., by hydrogenolysis or treatment with boron trichloride in the case where $P_1$ is benzyl, or by treatment with fluoride ion in the case where $P_1$ is a silyl group).

Alternatively, this compound of formula 1 can be prepared from a compound of formula 1 wherein $R_1$ is

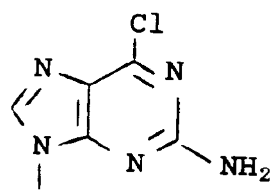

and $R_6$ is $-CH_2OH$ and $R_7$ is hydrogen by methods known in the art (e.g., treatment with hot methanolic ammonia).

Alternatively, this compound of formula 1 can be prepared by reacting an optionally protected compound of formula

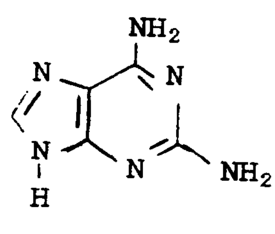

11

with a compound of formula 2 according to procedures analogous to those used in the preparation of a compound of formula 4, followed by removal of the protecting groups by methods known in the art. An optionally protected form of 11 can be protected at the amino ($-NH_2$) group by such exemplary groups as acyl, trityl or substituted trityl.

A compound of formula 1 wherein $R_1$ is

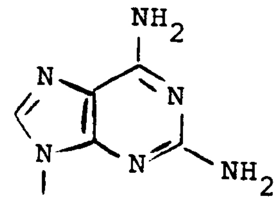

$R_6$ is -OH and $R_7$ is hydrogen can be prepared from a compound of formula 9 by methods known in the art, for example, by treatment with hot methanolic ammonia followed by removal of the benzyl protecting group (e.g., by hydrogenolysis or treatment with boron trichloride).

Alternatively, this compound of formula 1 can be prepared from a compound of formula 1 wherein $R_1$ is

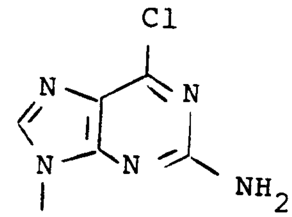

$R_6$ is -OH and $R_7$ is hydrogen by methods known in the art (e.g., treatment with hot methanolic ammonia).

Alternatively, this compound of formula 1 can be prepared by reacting an optionally protected compound of formula 11 with the compound of formula 5 according to procedures analogous to those used in the preparation of a compound of formula 6 followed by removal of the protecting groups by methods known in the art. An optionally protected compound of formula 11 can be protected at the amino ($-NH_2$) group by such exemplary groups as acyl, trityl, or substituted trityl.

A compound of formula 1 wherein $R_1$ is

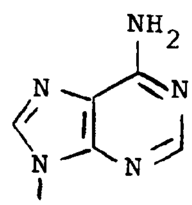

$R_6$ is-$CH_2OH$ and $R_7$ is hydrogen can be prepared by reacting a compound of formula

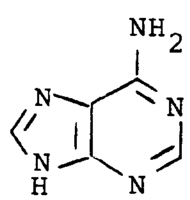

12

with a compound of formula 2 according to procedures analogous to those used in the preparation of a compound of formula 4, followed by removal of the protecting groups by methods known in the art.

Alternatively, this compound of formula 1 can be prepared by reacting a compound of formula

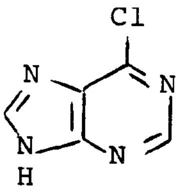

13

with a compound of formula 2 by methods analogous to those used in the preparation of compound 4. This affords the corresponding compound of formula

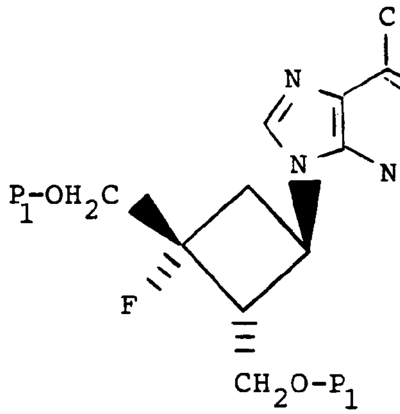

.

14

Treatment of a compound of formula 14 with hot ammonia in methanol or ethanol and subsequent removal of the $P_1$ hydroxy protecting groups yields the corresponding compound of formula 1 wherein $R_1$ is

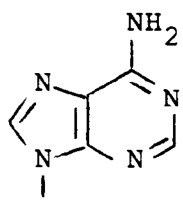

,

$R_6$ is $-CH_2OH$, and $R_7$ is hydrogen.

A compound of formula 1 wherein $R_1$ is

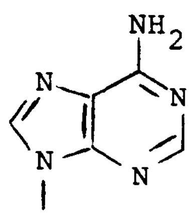

,

$R_6$ is -OH and $R_7$ is hydrogen can be prepared by reacting a compound of formula 12 with the compound of formula 7 according to the procedures analogous to those used in the preparation of a compound of formula 6 followed by removal of the protecting groups by methods known in the art.

Alternatively, this compound of formula 1 can be prepared by reaction of a compound of formula 13 with the compound of formula 5 by methods analogous to those used in the preparation of a compound of formula 8. This affords the corresponding compound of formula

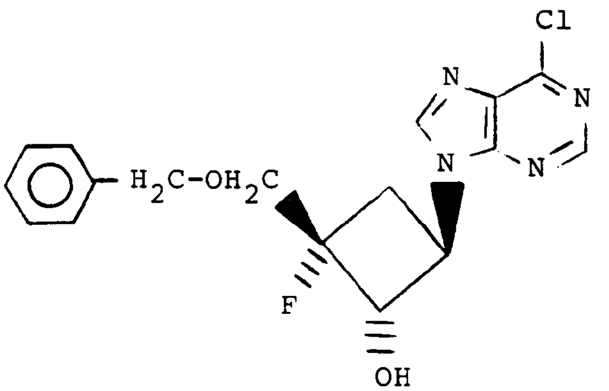

.

15

Treatment of a compound of formula 15 with hot ammonia in methanol or ethanol and subsequent removal of the benzyl protecting group yields the corresponding compound of formula 1 wherein $R_1$ is

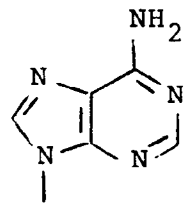

,

$R_6$ is -OH, and $R_7$ is hydrogen.

A compound of formula 1 wherein $R_1$ is

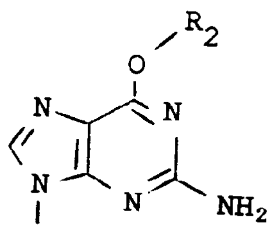

$R_6$ is $-CH_2OH$ and $R_7$ is hydrogen can be prepared from a compound of formula 8 or from a compound of formula 1 wherein $R_1$ is

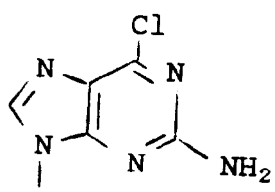

$R_6$ is $-CH_2OH$ and $R_7$ is hydrogen by methods known in the art. See, for example, J.F. Gerster, et al., J. Amer. Chem. Soc., 87, 3752 (1965); K.K. Ogilvie, et al., Can. J. Chem., 62, 2702 (1984); M.R. Harnden, et al. J. Med. Chem., 30, 1636 (1987).

Alternatively, this compound of formula 1 can be prepared by reacting a compound of formula

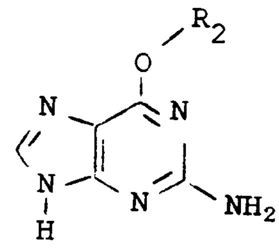

16

with a compound of formula 2 according to procedures analogous to those used in the preparation of a compound of formula 4, followed by removal of the protecting groups P by methods known in the art. The compound of formula 16 can be prepared from the compound of formula 7 by methods known in the art. See, for example, W.A. Bowles, et al., J. Med. Chem., 6, 471 (1963); M. MacCoss, et al., Tetrahedron Lett., 1815 (1985).

Similarly, a compound of formula 1 wherein $R_1$ is

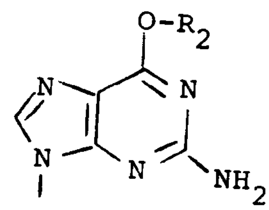

$R_6$ is -OH and $R_7$ is hydrogen can be prepared from a compound of formula 1 wherein $R_1$ is

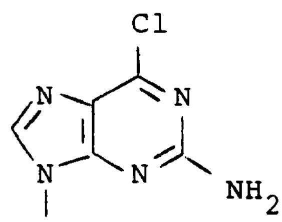

$R_6$ is -OH and $R_7$ is hydrogen by methods known in the art. Alternatively, this compound of formula 1 can be prepared from the compound of formula 9 by methods known in the art, followed by removal of the benzyl protecting groups by methods known in the art.

In another alternative, this compound of formula 1 can be prepared by reacting a compound of formula 16 with the compound of formula 5 according to procedures analogous to those employed in the preparation of the compound of formula 6, followed by removal of the benzyl protecting group by methods known in the art.

Compounds of formula 1 wherein $R_1$ is

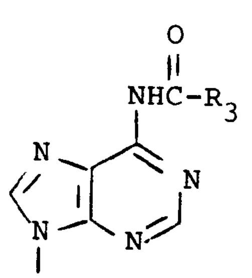

or

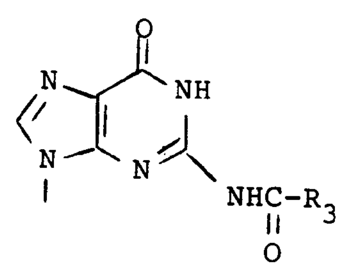

can be prepared from the correcponding compounds of formula 1 wherein $R_1$ is

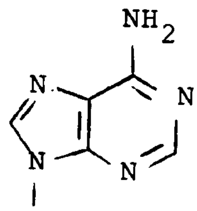

or

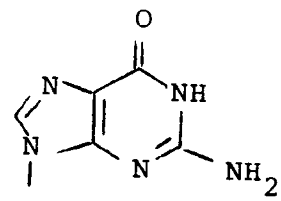

by methods known in the art. See, for example, C.B. Reese, Tetrahedron, 34, 3143 (1978); F. Benseler, et al., Synthesis, 45 (1986); G.S. Ti et al., J. Amer. Chem. Soc., 104, 1316 (1982).

Compounds of formula 1 wherein $R_1$ is

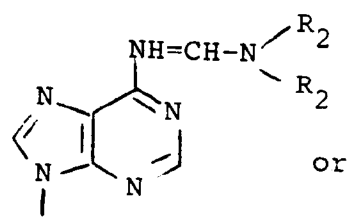

or

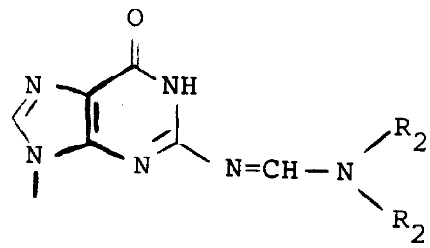

can be prepared from the corresponding compounds of formula 1 wherein $R_1$ is

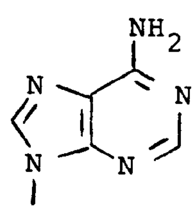

or

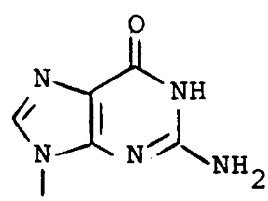

by procedures known in the art. See, for example, A. Holy and J. Zemlicka, Collect. Czech. Chem. Commun., 32, 3159(1967); K.K. Ogilvie, et al., Nucleosides and Nucleotides, 4, 507 (1985); M.H. Caruthers, et al., J. Amer. Chem. Soc., 108, 2040 (1986).

A compound of formula 1 wherein $R_1$ is

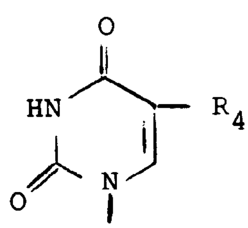

$R_4$ is hydrogen, fluoro, methyl, ethyl, n-propyl, 2-chloroethyl, or 2-fluoroethyl, $R_6$ is $-CH_2OH$, and $R_7$ is hydrogen can be prepared by reaction of the corresponding compound of formula

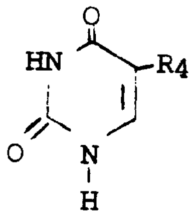

17

with a compound of formula 2 in the presence of a base such as potassium carbonate, sodium hydride, lithium hydride, or potassium hydride, in an aprotic polar solvent (e.g., dimethylformamide, dimethylsulfoxide, or sulfolane), in the optional presence of 18-crown-6 or 15-crown-5, to yield an intermediate of formula

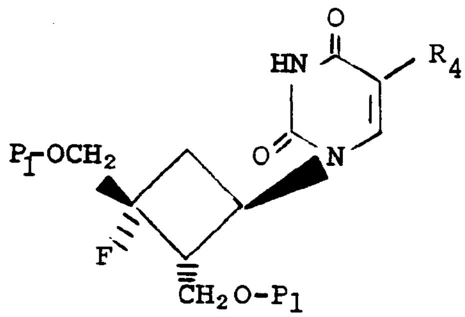

18

Alternatively, treatment of a compound of formula 2 with a mono-tetraalkylammonium salt of a compound of formula 17 in an aprotic polar solvent such as dimethylformamide, dimethylsulfoxide or sulfolane affords a compound of formula 18. A mono-tetraalkylammonium salt of a compound of formula 17 can be prepared by treatment of a compound of formula 17 (in an aprotic polar solvent such as dimethylformamide) with one equivalent of a tetraalkylammonium hydroxide, such as aqueous tetra-n-butylammonium hydroxide, and subsequent azeotropic removal of water.

Removal of the protecting groups $P_1$ from a compound of formula 18 provides the corresponding compound of formula 1 wherein $R_1$ is

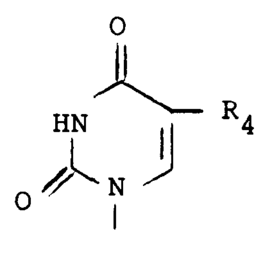

$R_4$ is hydrogen, fluoro, methyl, ethyl, n-propyl, 2-chloroethyl, or 2-fluoroethyl, $R_6$ is $-CH_2OH$, and $R_7$ is hydrogen. For example, when $P_1$ is a silyl group, deprotection can be accomplished with fluoride ion. When $P_1$ is a benzyl group, deprotection can be accomplished by hydrogenolysis (e.g., palladium hydroxide on carbon in cyclohexene and ethanol) or by treatment with boron trichloride.

The compound of formula 17 wherein $R_4$ is 2-chloroethyl or 2-fluoroethyl can be prepared by methods known in the art [H. Griengl, et al., J. Med. Chem., 30, 1199 (1987); J. Med. Chem., 28, 1679 (1985)].

Similarly, the compounds of formula 1 wherein

$R_1$ is

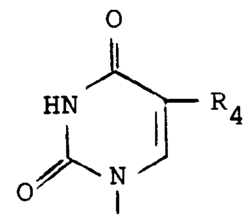

$R_4$ is hydrogen, fluoro, methyl, ethyl, n-propyl, 2-chloroethyl or 2-fluoroethyl, $R_6$ is -OH and $R_7$ is hydrogen can be prepared by reacting a compound of formula 17 with the compound of formula 5 in the presence of a base such as potassium carbonate, sodium hydride, lithium hydride, or potassium hydride, in an aprotic polar solvent (e.g., dimethylformamide, dimethylsulfoxide or sulfolane), in the optional presence of 18-crown-6 or 15-crown-5, to yield an intermediate of formula

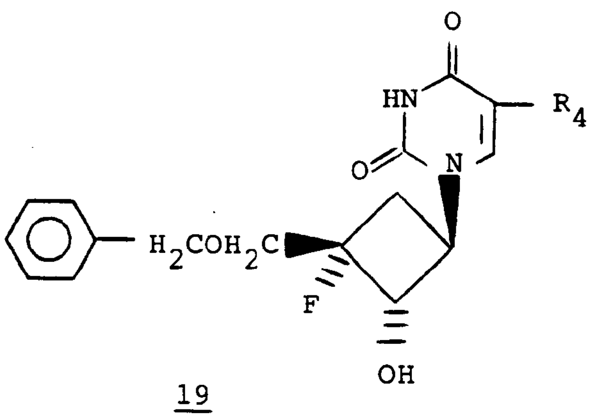

.

19

Alternatively, treatment of compound of formula 2 with a mono-tetraalkylammonium salt of a compound of formula 17 in an aprotic polar solvent such as dimethyl formamide, dimethylsulfoxide, or sulfolane affords a compound of formula 19.

Removal of the benzyl protecting group from the compound of formula 19 by hydrogenolysis or by treatment with boron trichloride provides a compound of formula 1 wherein $R_1$ is

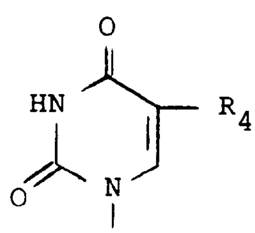

,

$R_4$ is hydrogen, fluoro, methyl, ethyl, n-propyl, 2-chloroethyl, or 2-fluoroethyl, $R_6$ is -OH, and $R_7$ is hydrogen.

The compounds of formula 1 wherein $R_1$ is

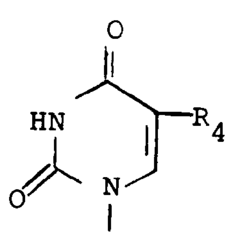

,

$R_4$ is fluoro, $R_6$ is -OH or $-CH_2OH$, and $R_7$ is hydrogen can also be prepared from the corresponding compound

wherein $R_4$ is hydrogen by fluorination with trifluoromethyl hypofluorite using methodology known in the art. Optionally, the hydroxyl groups can be protected, for example, by acyl groups. After fluorination, deprotection using methanolic ammonia or aqueous hydroxide affords the desired product. For example, see M.J. Robins, et al., J. Amer. Chem. Soc., 93, 5277 (1971) and Chem. Commun., 18 (1972); T.S. Lin, et al. J. Med. Chem., 26, 1691 (1983).

The compounds of formula 1 wherein $R_1$ is

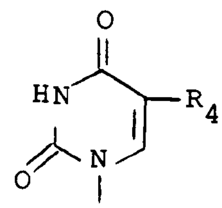

,

$R_4$ is 2-chloroethyl or 2-fluoroethyl,
$R_6$ is $-CH_2OH$ and $R_7$ is hydrogen can also be prepared from a compound of formula

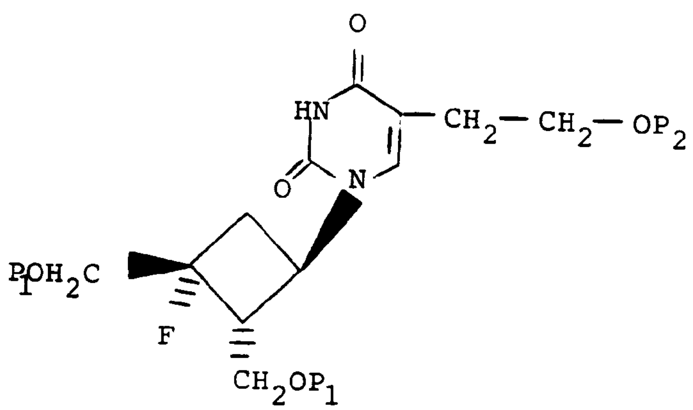

20

wherein $P_2$ and $P_1$ are different protecting groups wherein $P_2$ can be selectively removed in the presence of $P_1$. For example, when $P_2$ is a silyl, trityl or substituted trityl group, $P_1$ can be a benzyl group. Similarly, when $P_2$ is an acyl or benzyl group, $P_1$ can be a silyl protecting group. Selective removal of the protecting group $P_2$ yields a compound of formula 18 wherein $R_4$ is 2-hydroxyethyl. Treatment of this compound with triphenylphosphine-carbon tetrachloride and subsequent removal of protecting groups $P_1$ affords the compound of formula 1 wherein $R_4$ is 2-chloroethyl. Similar treatment using triphenylphosphine-N-bromosuccinimide or triphenylphosphine N-bromosuccinimide-tetrabutylammonium iodide in place of triphenylphosphine-carbon tetrachloride (e.g., see H. Griengl, et al., J. Med. Chem., 28, 1679 (1985)) affords compounds of formula 20 wherein $R_4$ is 2-bromoethyl or 2-iodoethyl, respectively. Subsequent treatment with fluoride ion, followed by removal of protecting groups P, provides the compound of formula 1 wherein $R_4$ is 2-fluoroethyl. When $P_1$ is a silyl group, deprotection will occur upon treatment with fluoride ion. Alternatively, treatment of a compound of formula 18 wherein $R_4$ is 2-hydroxyethyl, with diethylaminosulfur trifluoride provides, upon removal of the protecting groups $P_1$, a compound of formula 1 wherein $R_4$ is 2-fluoroethyl.

The compound of formula 20 can be prepared by reaction of a compound of formula

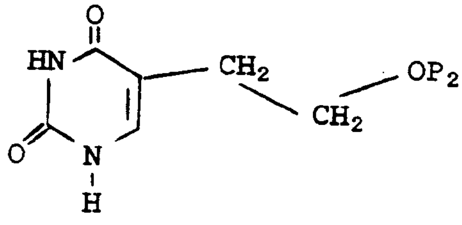

21

with a compound of formula 2 by methods analogous to those used for the preparation of 18 wherein, for example, $R_4$ is hydrogen, methyl or ethyl. The compound of formula 21 can be prepared from the corresponding free alcohol by methods known in the art.

Similarly, the compounds of formula 1 wherein $R_1$ is

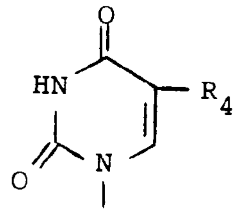

,

$R_4$ is 2-chloroethyl or 2-fluoroethyl, $R_6$ is -OH and $R_7$ is hydrogen can be prepared as described above from a compound of formula

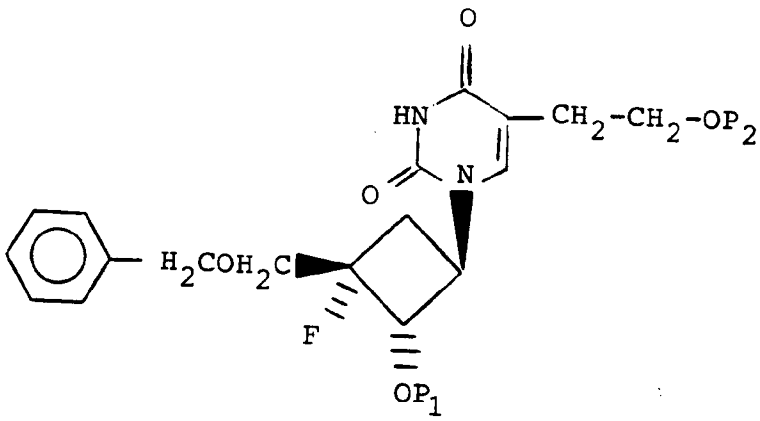

22

wherein $P_2$ and $P_1$ are protecting groups chosen so that $P_2$ can be selectively removed in the presence of $P_1$ and the benzyl protecting group. For example, when $P_2$ is silyl, trityl, or substituted trityl group, $P_1$ can be an acyl group such as acetyl or benzoyl. This compound of formula 22 can be prepared by acylation of the product formed by the reaction of a compound of formula 21 with the compound of formula 5 by methods analogous to those used for the preparation of 19 wherein, for example, $R_4$ is hydrogen, methyl, or ethyl.

The compounds of formula 1 wherein $R_1$ is

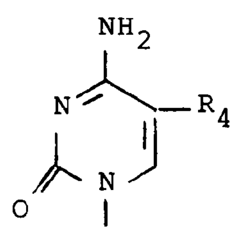

,

$R_4$ is hydrogen, fluoro, methyl, ethyl, n-propyl, 2-chloroethyl, or 2-fluoroethyl, $R_6$ is -$CH_2OH$ or -OH, and $R_7$ is hydrogen can be prepared from the corresponding compounds of formulas 18 or 19 by methods known in the art. See, for example, I. Wempner, et al., in "Synthetic Procedures in Nucleic Acid Chemistry", Vol. 1, W.W. Zorbach and R.S. Tipson, Eds., Interscience Publishers, N.Y., p. 299, 1968; T.S. Lin, et al., J. Med. Chem., 26, 1691 (1983); P. Herdewijn, et al., J. Med. Chem., 28, 550 (1985). Deprotection yields the corresponding compound of formula 1.

Alternatively, the compound of formula 1, wherein $R_1$ is

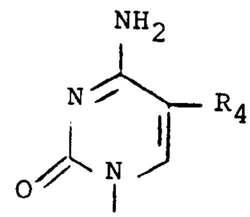

,

$R_4$ is hydrogen, fluoro, methyl, ethyl, n-propyl, 2-chloroethyl, or 2-fluoroethyl, $R_6$ is $-CH_2OH$, and $R_7$ is hydrogen can be prepared by reaction of the corresponding compound of formula

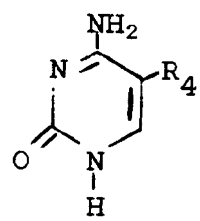

23

with a compound of formula 2 in the presence of a base such as potassium carbonate, sodium hydride, or potassium hydride in an aprotic solvent (e.g., dimethylformamide, dimethylsulfoxide, or sulfolane), in the optional presence of 18-crown-6 or 15-crown-5 to yield an intermediate of formula

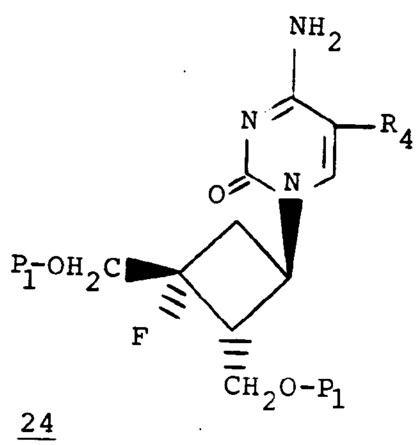

24

.

Removal of the protecting groups yields the compound of formula 1 wherein $R_1$ is

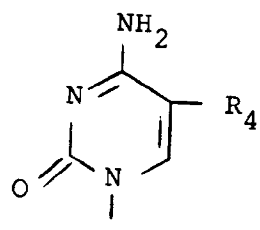

,

$R_4$ is hydrogen, fluoro, methyl, ethyl, n-propyl, 2-chloroethyl, or 2-fluoroethyl, $R_6$ is $-CH_2OH$, and $R_7$ is hydro-

gen.

Alternatively, treatment of a compound of formula 2 with a tetraalkylammonium salt of a compound of formula 23 in an aprotic polar solvent such as dimethylformamide, dimethylsulfoxide or sulfolane provides a compound of formula 24 A tetraalkylammonium salt of a compound of formula 23 can be prepared by treatment of a compound of formula 23 (in an aprotic polar solvent such as dimethylformamide) with a tetraalkylammonium hydroxide such as aqueous tetra-n-butylammonium hydroxide and subsequent azeotropic removal of water.

Similarly, the compounds of formula 1 wherein $R_1$ is

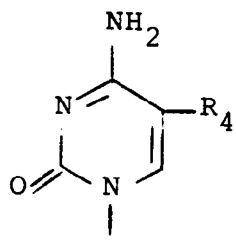

,

$R_4$ is hydrogen, fluoro, methyl, ethyl, n-propyl, 2-chloroethyl, or 2-fluoroethyl, $R_6$ is -OH, and $R_7$ is hydrogen can be prepared by reacting the corresponding compound of formula 23 with a compound of formula 5 in the presence of a base such as potassium carbonate, sodium hydride, or potassium hydride in an aprotic solvent such as dimethylformamide, dimethylsulfoxide, or sulfolane and the optional presence of 18-crown-6 or 15-crown-5 to yield the intermediate of the formula

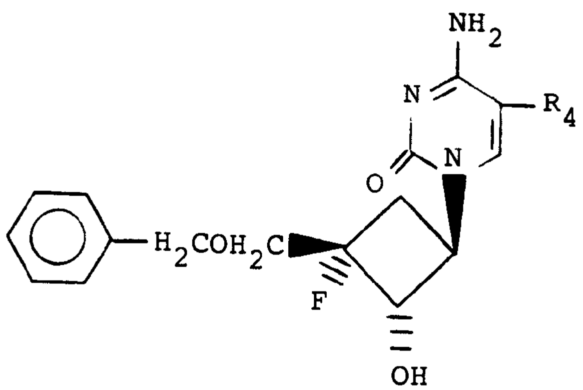

.

25

Removal of the benzyl protecting group yields the compound of formula 1 wherein $R_1$ is

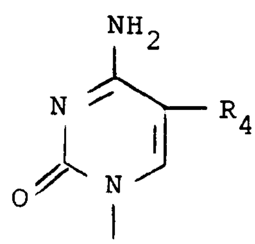

,

$R_4$ is hydrogen, fluoro, methyl, ethyl, n-propyl, 2-chloromethyl, or 2-fluoroethyl, $R_6$ is -OH and $R_7$ is hydrogen.

Alternatively, treatment of a compound of formula 5 with a tetraalkylammonium salt of a compound of formula 23 in an aprotic polar solvent such as dimethylformamide, dimethylsulfoxide, or sulfolane provides a compound of formula 25.

Alternatively, the compound of formula 1 wherein $R_1$ is

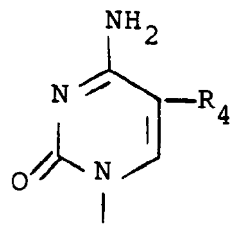

$R_4$ is fluoro, $R_6$ is $-CH_2OH$ or -OH, and $R_7$ is hydrogen can be prepared from the corresponding compound wherein $R_1$ is

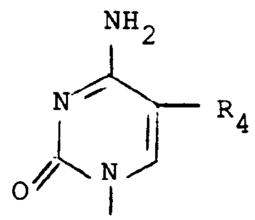

,

$R_4$ is hydrogen, $R_6$ is $-CH_2OH$ or -OH, and $R_7$ is hydrogen by fluorination with trifluoromethyl hypofluorite using methodology known in the art. Optionally, the hydroxyl and/or amino groups can be protected, for example, by acyl groups. After fluorination, deprotection using methanolic ammonia or aqueous hydroxide affords the desired products. See, for example, M.J. Robins, et al., J. Amer. Chem. Soc., 93, 5277 (1971) and Chem. Commun., 18 (1972); T.S. Lin, et al., J. Med. Chem., 26, 1691 (1983).

The compounds of formula 1 wherein $R_1$ is

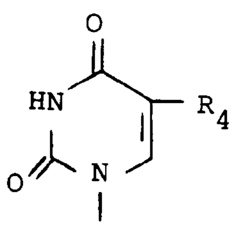

or

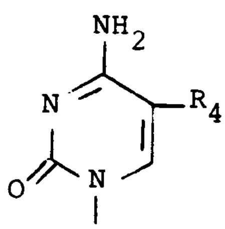

,

$R_4$ is chloro, bromo or iodo, $R_6$ is $-CH_2OH$ or -OH, and $R_7$ is hydrogen can be prepared from the corresponding compounds of formla 1 wherein $R_4$ is hydrogen by methods known in the art. See, for example, "Basic Principals in Nucleic Acid Chemistry", Vol. 1, P.O.P. Ts'O, Ed., Academic Press, N.Y., p. 146, 1974; P.K. Chang in "Nucleic Acid Chemistry" Part 3, L.B. Townsend and R.S. Tipson, Eds., John Wiley and Sons, N.Y., p.46, 1986.

The compounds of formula 1 wherein $R_1$ is

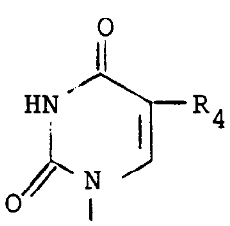

or

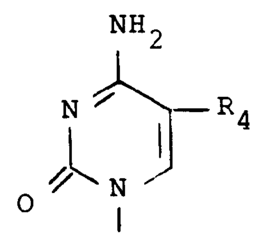

,

wherein $R_4$ is trifluoromethyl, $R_6$ is $-CH_2OH$ or -OH, and $R_7$ is hydrogen can be prepared from the corresponding compounds of formula 1 wherein $R_4$ is iodo and the hydroxy and amino ($-NH_2$) groups are protected, for example, by acyl groups, by treatment with trifluoromethyl iodide and copper according to procedures known in the art. Subsequent deprotection using methanolic ammonia or sodium methoxide in methanol yields the desired compound of formula 1 wherein $R_4$ is trifluoromethyl, $R_6$ is $-CH_2OH$ or -OH, and $R_7$ is hydrogen. See, for

example, Y. Kobayashi, et al., J. Chem. Soc. Perkin 1, 2755 (1980); S. Lin, et al., J. Med. Chem., 26, 1691 (1983).

The compounds of formula 1 wherein $R_1$ is

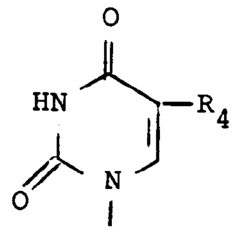 or 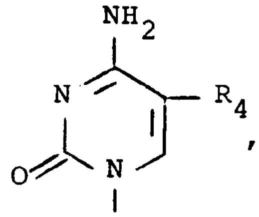 ,

$R_4$ is

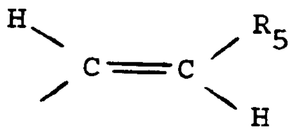 ,

$R_5$ is chloro, bromo, iodo, hydrogen, methyl or trifluoromethyl, $R_6$ is $-CH_2OH$ or -OH, and $R_7$ is hydrogen can be prepared from the corresponding compounds of formula 1 wherein $R_4$ is iodo or -HgCl via organopalladium intermediates. These compounds of formula 1 wherein $R_4$ is -HgCl can be prepared from the corresponding compounds of formula 1 wherein $R_4$ is hydrogen by methods known in the art. See, for example, M. Ashwell, et al., Tetrahedron, 43, 4601 (1987); M.E. Perlman, et al., J. Med. Chem., 28, 741 (1985); P. Herdewijn, et al., J. Med. Chem., 28, 550 (1985); D.E. Bergstrom, et al., J. Med. Chem., 27, 279 (1984); and references in E. DeClercq, et al., Pharmac. Ther., 26, 1 (1984).

Compounds of formula 1 wherein $R_5$ is

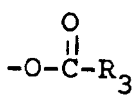 or 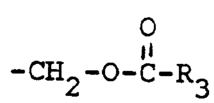 ,

and/or $R_7$ is

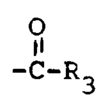

can be prepared by methods known in the art from the corresponding compounds of formula 1 wherein $R_6$ is -OH or $-CH_2OH$ and $R_7$ is hydrogen. For examples of such acylation procedures see:
"Synthetic Procedures in Nucleic Acid Chemistry", Vol. 1, W. W. Zorbach and R. S. Tipson, Eds., John Wiley and Sons, 1968; "Nucleic Acid Chemistry," Part 1, L.B. Townsend and R. S. Tipson, Eds., John Wiley and Sons, 1978; Y. Ishido, et al., Nucleosides and Nucleotides, 5, 159 (1986); J.C. Martin, et al., J. Pharm. Sci., 76, 180 (1987); A. Matsuda, et al. Synthesis, 385 (1986).

Compounds of formula 1 wherein $R_6$ is $-OPO_3H_2$ or $-CH_2-OPO_3H_2$ and $R_7$ is $PO_3H_2$ can be prepared from the corresponding compounds of formula 1 wherein $R_5$ is -OH or $-CH_2-OH$ and $R_7$ is hydrogen by procedures known in the art. See, for example, H. Schaller, et al., J. Amer. Chem. Soc., 85, 3821 (1963); J. Beres, et al., J. Med. Chem., 29, 494 (1986); R. Noyori, et al., Tetrahedron Lett., 28, 2259 (1987); W. Pfeiderer, et al., Helv. Chim. Acta., 70, 1286 (1987); "Nucleic Acid Chemistry". Part 2, L.B. Townsend and R.S. Tipson, Eds., John Wiley and Sons, 1978.

The intermediates of formula 2 can be prepared according to the following procedure. A cyclobutane compound of formula 26 [prepared as described by Slusarchyk et al., Tetrahedron Letters, 30, 6453 (1989) and

European Patent Application No. 335,355] is regioselectively saponified using, for example, aqueous lithium hydroxide in tetrahydrofuran and ethanol to give the compound of formula 27. The ester group of the compound of formula 27 is chemoselectively reduced using a reducing agent such as lithium triethylborohydride or lithium borohydride to give the compound of formula 28. The carboxyl group of the compound of formula 28 is esterified with an alkyl group $R_8$ such as methyl or ethyl by known procedures to yield the compound of formula 29. The hydroxyl group of the compound of formula 29 is protected with a protecting group $P_1$ (benzyl or silyl) by methods known in the art to give a compound of formula 30. Treatment of a compound of formula 30 with an amide base such as lithium diisopropylamide followed by the addition of a fluorinating agent such as perchloryl fluoride [see, for example, C.L.J.Wang, et al. Chem. Communications, 48(1976)] or other fluorinating agents such as acetyl hypofluorite, N-fluoroalkylsulfonamides or N-fluoroalkylsulfonamides or N-fluoro-O-benzenedisulfonimide [see, for example, S. Rosen et al., Synthesis, 665(1985); W.E. Barnette, J. Amer. Chem. Soc., 106, 452 (1984); F.A. Davis et al., Tetrahedron Letters, 1631 (1991)] provides the compounds of formulas 31 and 32 which can be separated by column chromatography.

Reduction of a compound of formula 31 with a reducing agent such as diisobutylaluminum hydride, lithium aluminum hydride, or lithium borohydride provides the compound of formula 33. The hydroxyl group of a compound of formula 33 can be protected with a protecting group $P_1$ by methods known in the art. Hydrolysis of a compound of formula 34 using, for example, aqueous sulfuric acid in acetonitrile or p-toluenesulfonic acid in acetone provides a compound of formula 35. Treatment of a compound of formula 35 with a reducing agent such as lithium trisiamylborohydride or lithium tri-sec-butylborohydride, followed by aqueous hydrogen peroxide and sodium bicarbonate, provides a compound of formula 36.

A compound of formula 36 can be converted to the intermediate of formula 2 by methods known in the art. For example, treatment of a compound of formula 36 with p-toluenesulfonyl chloride or methanesulfonyl chloride in pyridine yields a compound of formula 2 wherein X is p-toluenesulfonyloxy or methanesulfonyloxy, respectively.

The above description is shown in the following schematic:

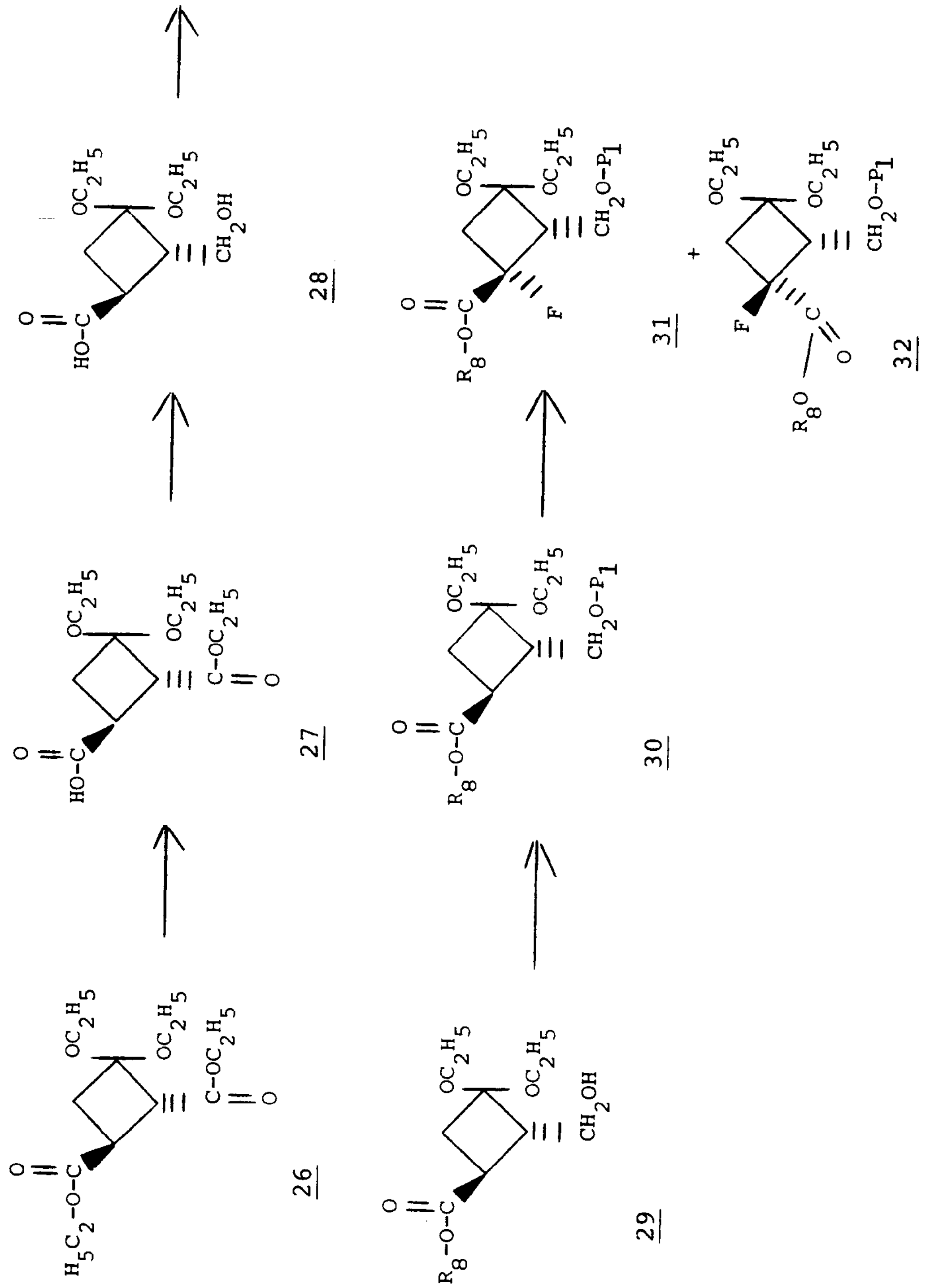

26 27 28 29 30 31 32

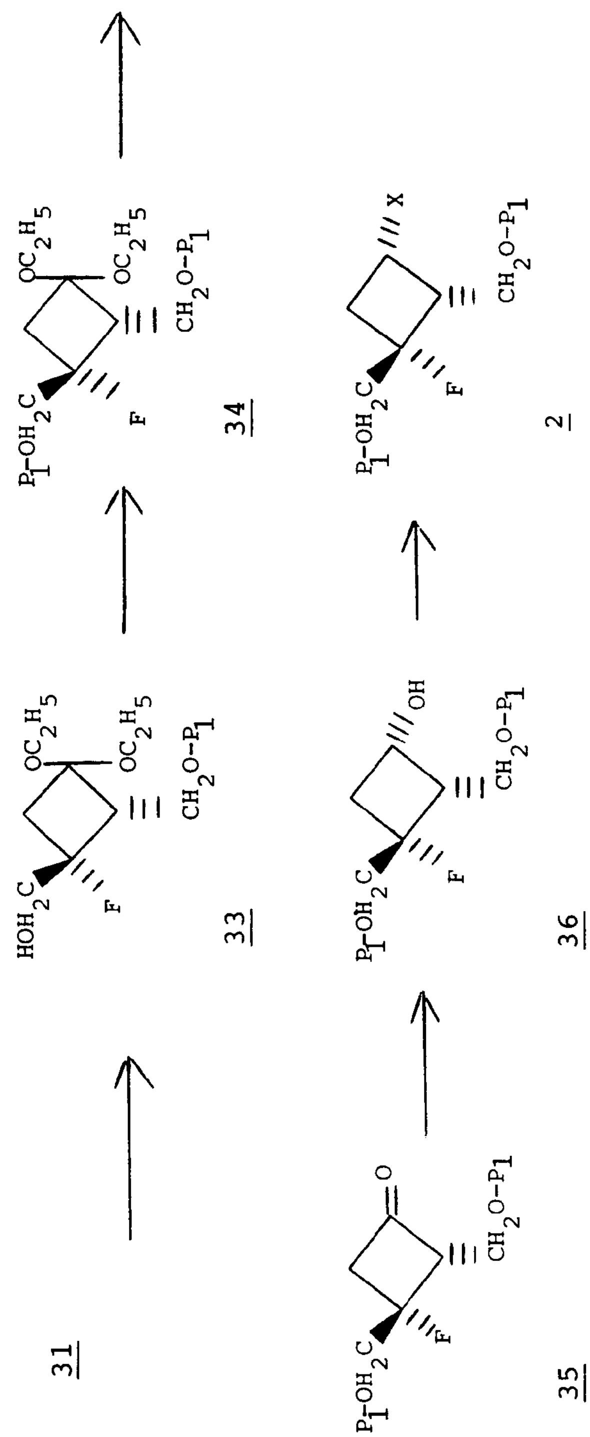

31 33 34

35 36 2

The intermediate of formula 5 can be prepared according to the following procedure. Diethyl fluoromalonate 38 [see N. Ishikawa et al., J. Fluorine Chem., 25, 203 (1984); N. Ishikawa et al., Chem. Letters, 107 (1981); T. Fuchikami et al., Chem. Letters, 1573 (1984); F.L.M. Pattison et al., J. Chem. Soc., 3280 (1959); R.L. Buchanan et al., Can. J. Chem., 43, 1700(1965)] can be converted to the compound of formula 39 by treatment with a hydride base, such as sodium hydride or potassium hydride, followed by the addition of benzyl chloromethyl ether.

Treatment of the compound of formula 39 with a reducing agent such as lithium borohydride, lithium aluminum hydride, diisobutylaluminum hydride, or lithium triethylborohydride provides the compound of formula 40. The hydroxyl substituents of the compound of formula 40 can be converted to suitable leaving groups Y, wherein Y, for example, is trifluoromethanesulfonyloxy, by methods known in the art to provide a compound of formula 41.

Treatment of a compound of formula 41 with the lithium salt of methyl methylsulfinylmethyl sulfide provides the compound of formula 42 as a mixture of diastereomers. [See, for example, K. Ogura et al., Bull. Chem. Soc. Japan, 57, 1637(1984); K. Ogura et al., Chem Letters, 813(1982); K. Ogura et al., Tetrahedron Letters 3653(1974); K. Ogura et al., Tetrahedron Letters, 3151(1971)].

The compound of formula 42 can be converted to the compound of formula 43 by treatment with trimethylorthoformate and methanol in the presence of an acid catalyst such as sulfuric acid [See K. Ogura et al., Tetrahedron Letters, 2681(1972)]. Acid hydrolysis of the compound of formula 43 using, for example, aqueous sulfuric acid in acetonitrile or p-toluenesulfonic acid in acetone provides the compound of formula 44. Alternatively, the compound of formula 44 can be obtained directly from a compound of formula 42 by acid hydrolysis using, for example, aqueous sulfuric, perchloric, or hydrochloric acid [See K. Ogura et al., Bull. Chem. Soc. Japan, 57, 1637(1984); K. Ogura et al., Chem. Letters, 813(1982)].

Treatment of the compound of formula 44 with a reducing agent such as lithium trisiamylborohydride provides the compound of formula 45 as a mixture of diastereomers. Alternatively, other reducing agents such as sodium borohydride, lithium borohydride, lithium aluminum hydride, lithium tri-sec-butylborohydride, or diisobutylaluminum hydride can be used.

The compound of formula 45 can be converted to a compound of formula 46 wherein X' is a leaving group such as an alkyl, phenyl, substituted alkyl, or substituted phenyl sulfonate (e.g., p-toluenesulfonyloxy) by methods known in the art. Basic elimination of a sulfonic acid from a compound of formula 46 using a base such as potassium t-butoxide or lithium diisopropylamide in an aprotic solvent, such as dimethylsulfoxide or tetrahydrofuran, yields the compound of formula 47.

Epoxidation of the compound of formula 47 using a peracid, such as m-chloroperoxybenzoic acid, yields the mixture of the compounds of formulas 5 and 48 which can be separated by chromatography.

This process description is shown in the following schematic:

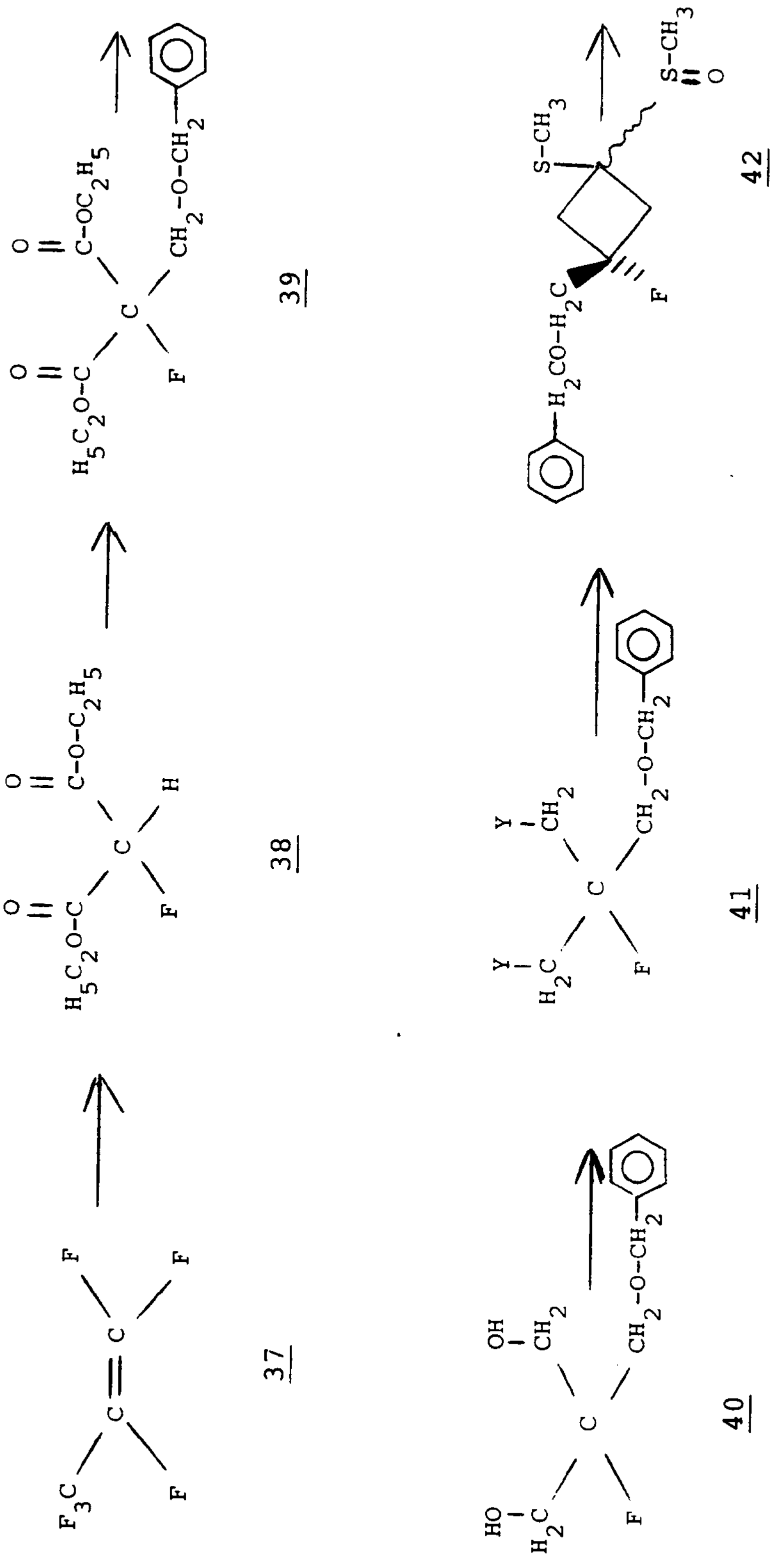

37

38

39

40

41

42

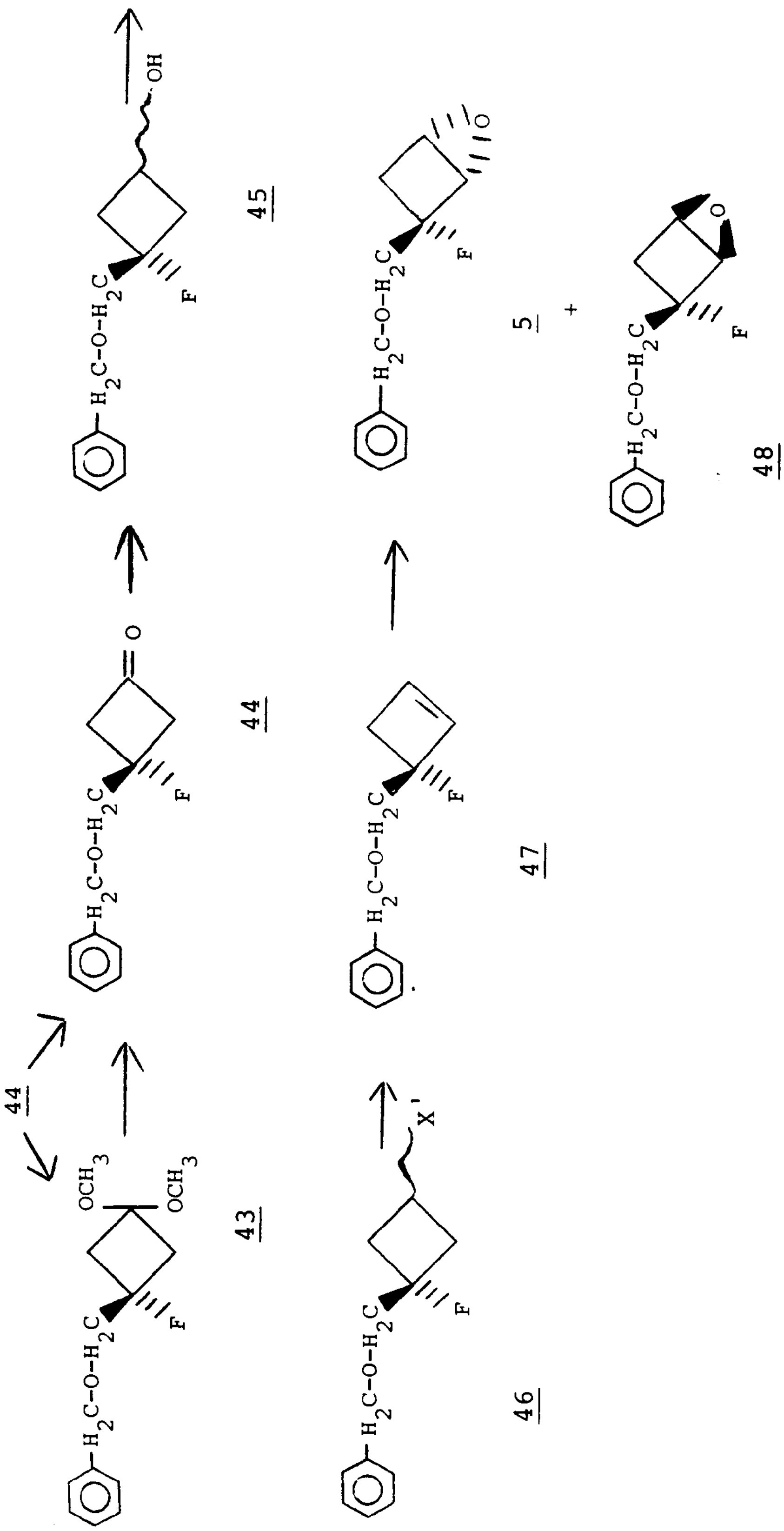

45

5

48

44

47

43

46

Unless indicated otherwise, the stereochemistry shown for the compounds of formula 1 including the intermediates leading thereto is relative, not absolute. It is drawn to show that in these compounds the base represented by $R_1$ is trans to the vicinal substituent, and $R_1$ is trans to the fluorine atom. The compounds of formula 1 can exist as levorotatory or dextrorotatory enantiomers or as mixtures thereof. All of these forms

are within the scope of this invention.

For example, the 1R-enantiomer of the compound of formula 1 wherein $R_1$ is

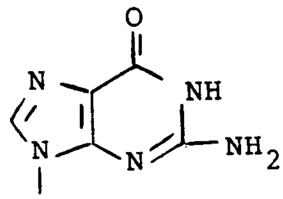

and $R_6$ is $-CH_2OH$ and $R_7$ is hydrogen, i.e., [1R-(1α,2β,3β)]-2-amino-9-[3-fluoro-2,3-bis(hydroxymethyl)-cyclobutyl]-1,9-dihydro-6H-purin-6-one, can be prepared from the 1S-enantiomer of the compound of formula 2 wherein X is p-toluenesulfonyloxy and P is benzyl, i.e., [1S-(1α,2α,3α)]-3-fluoro-2,3-bis[(phenylmethoxy)methyl]cyclobutanol, 4-methylbenzenesulfonate ester, using methods analogous to those used in the preparation of the corresponding racemic compound of formula 1. Reaction of [1S-(1α,2α,3α)]-3-fluoro-2,3-bis[(phenylmethoxy)methyl]cyclobutanol, 4-methylbenzenesulfonate ester with a compound of formula 3 in the presence of a base, such as potassium carbonate, in an aprotic polar solvent, such as dimethylformamide, and subsequent removal of the benzyl protecting groups yields [1R-(1α,2β,3β)]-2-amino-9-[3-fluoro-2,3-bis(hydroxymethyl)cyclobutyl]-1,9-dihydro-6H-purin-6-one.

[1S-(1α,2β,3β)]-2-Amino-9-[3-fluoro-2,3-bis (hydroxymethyl)cyclobutyl]-1,9-dihydro-6H-purin-6-one can be prepared in an analogous fashion from [1R-(1α,2α,3α)]-3-fluoro-2,3-bis[(phenylmethoxy)methyl]cyclobutanol, 4-methylbenzenesulfonate ester.

[1S-(1α,2α,3α)]-3-Fluoro-2,3-bis[(phenylmethoxy)methyl]cyclobutanol, 4-methylbenzenesulfonate ester can be prepared from the corresponding alcohol, i.e., [1S-(1α,2α,3α)]-3-fluoro-2,3-bis[(phenylmethoxy)methyl]cyclobutanol, by methods known in the art. [1S-(1α,2α,3α)]-3-Fluoro-2,3-bis[(phenylmethoxy)methyl]cyclobutanol can be prepared from (2S-cis)-3-fluoro-1,1-dimethoxy-2,3-bis-[(phenylmethoxy)methyl]cyclobutane by hydrolysis of the ketal with aqueous sulfuric acid in acetonitrile, and subsequent reduction of the resulting cyclobutanone with, for example, lithium trisiamylborohydride. (2S-cis)-3-Fluoro-1,1-dimethoxy-2,3-bis-[(phenylmethoxy)methyl]cyclobutane can be prepared from (1R-cis)-1-fluoro-3,3-dimethoxy-2-[(phenylmethoxy)methyl]cyclobutanecarboxylic acid, methyl ester by reduction of the ester group with, for example, diisobutylaluminum hydride, and subsequent benzylation of the resulting alcohol by methods known in the art. (1R-cis)-1-Fluoro-3,3-dimethoxy-2-[(phenylmethoxy)methyl]cyclobutanecarboxylic acid, methyl ester can be prepared from (1S-trans)-3,3-dimethoxy-2-[(phenylmethoxy)methyl]cyclobutanecarboxylic acid, methyl ester by treatment with an amide base, such as lithium diisopropylamide, followed by the addition of a fluorinating agent, such as perchloryl fluoride. (1S-trans)-3,3-Dimethoxy-2-[(phenylmethoxy)methyl]cyclobutanecarboxylic acid, methyl ester can be prepared from (1S-trans)-3,3-dimethoxy-2-(hydroxymethyl)cyclobutanecarboxylic acid by methyl esterification of the carboxyl group and subsequent benzylation of the resulting alcohol by methods known in the art. (1S-trans)-3,3-Dimethoxy-2-(hydroxymethyl)cyclobutanecarboxylic acid can be prepared from (1S-trans)-3,3-dimethoxy-1,2-cyclobutanedicarboxylic acid, diethyl ester by regioselective saponification of the C-1 ester group with, for example, lithium hydroxide in tetrahydrofuran, and subsequent reduction of the C-2 ester group with, for example, lithium triethylborohydride. (1S-trans)-3,3-Dimethoxy-1,2-cyclobutanedicarboxylic acid, diethyl ester can be prepared from (1S-trans)-3,3-dimethoxy-1, 2-cyclobutanedicarboxylic acid, di-(-)-menthyl ester by treatment with sodium ethoxide in ethanol. (1S-trans)-3,3-Dimethoxy-1,2-cyclobutanedicarboxylic acid, di-(-)-menthyl ester can be prepared by complexation of (-)-dimenthylfumerate with diisobutylaluminum chloride or diethylaluminum chloride at -78°C in toluene or toluene/hexane, followed by the addition of 1,1-dimethoxyethylene.

In an analogous manner, [1R-(1α,2α,3α)]-3-fluoro-2,3-bis-[(phenylmethoxy)methyl]cyclobutanol, 4-methylbenzenesulfonate ester can be prepared via the product resulting from the cycloaddition of (+)-dimenthylfumerate and 1,1-dimethoxyethylene.

The compounds of formula 1 wherein $R_1$ is

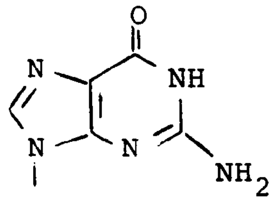,
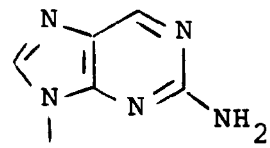,
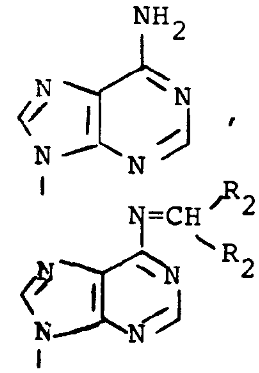,
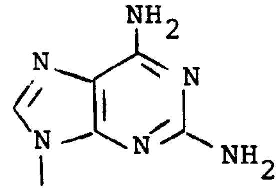,
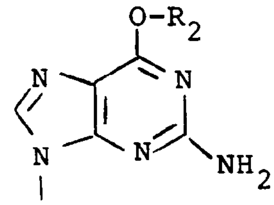,
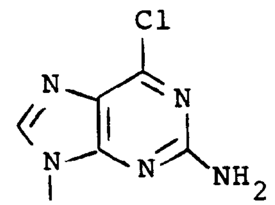,
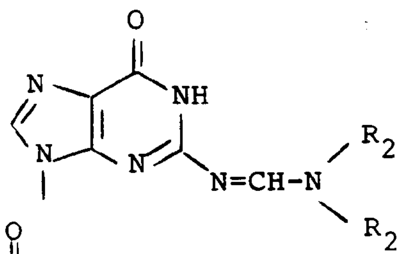,
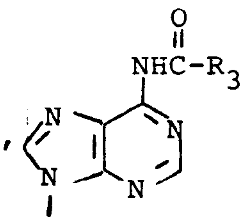,
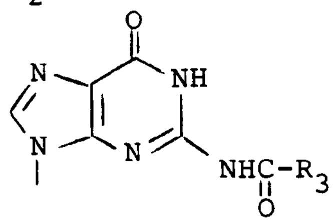, or
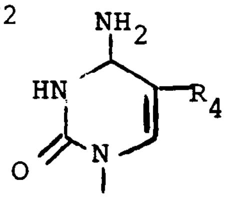

can form acid addition salts with inorganic or organic acids. Illustrative are the hydrohalide (e.g., hydrochloride and hydrobromide), alkylsulfonate, sulfate, phosphate, and carboxylate salts.

The compounds of formula 1 wherein $R_1$ is

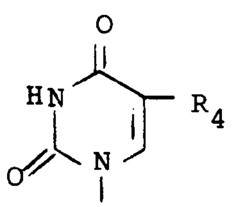,
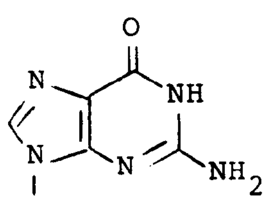,
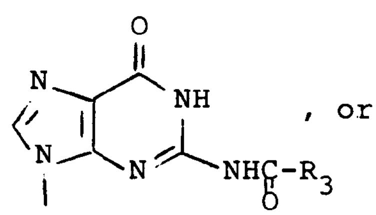, or
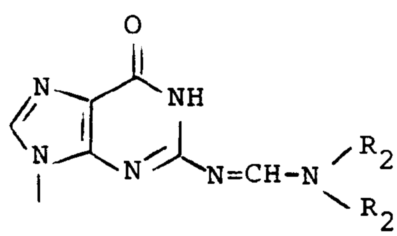

can form basic salts with inorganic and organic bases. Illustrative are alkali metal salts (e.g., sodium and potassium), alkaline earth metal salts (e.g., calcium and magnesium), ammonium and substituted ammonium salts.

The compounds of formula 1 wherein $R_6$ is $-CH_2-O-PO_3H_2$ or $-O-PO_3H_2$ and/or $R_7$ is $-PO_3H_2$ can form basic salts with inorganic and organic bases. Illustrative are the alkali metal salts (e.g., sodium and potassium), alkaline earth metal salts (e.g., calcium and magnesium), ammonium and substituted ammonium salts.

Preferred compounds of this invention are those of formula 1 wherein $R_1$ is

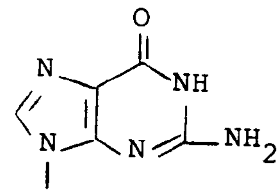

;

$R_6$ is -OH or -$CH_2OH$; and $R_7$ is hydrogen.

The most preferred compound is (1α,2β,3β)-2-amino-9-[3-fluoro-2,3-bis(hydroxymethyl)cyclobutyl]-1,9-dihydro-6H-purin-6-one.

This compound exists as the following two enantiomers: [1R-(1α,2β,3β)]-2-amino-9-[3-fluoro-2,3-bis(hydroxymethyl)cyclobutyl]-1,9-dihydro-6H-purin-6-one, which is preferred, and [1S-(1α,2β, 3β)]-2-amino-9-[3-fluoro-2,3-bis(hydroxymethyl)cyclobutyl]-1,9-dihydro-6H-purin-6-one.

The following examples are specific embodiments of this invention. Unless otherwise noted, the compounds exemplified have been prepared as racemic mixtures.

Example 1

(1α,2β,3β)-2-Amino-9-[3-fluoro-2,3-bis(hydroxymethyl)cyclobutyl]-1,9-dihydro-6H-purin-6-one

a) trans-3,3-Diethoxy-1,2-cyclobutanedicarboxylic acid, 2-ethyl ester

To a solution of trans-3,3-diethoxy-1,2-cyclobutanedicarboxylic acid, diethyl ester [5.0 g., 17.4 mmole, prepared as described by Slusarchyk et al., Tetrahedron Letters, 6453 (1989) and European Patent Application 335,355] in tetrahydrofuran (70 ml.) was added lithium hydroxide monohydrate (0.729 g., 17.4 mmole). Absolute ethanol (50 ml.) was added to the mixture until the turbidity disappeared. The resulting solution was stirred overnight. The organic solvents were rotary evaporated leaving an aqueous solution which was extracted with hexane (100 ml.). After the addition of sodium bisulfate (2.4 g., 17.4 mmole) and saturation with sodium chloride, the solution was extracted with ethyl acetate (3 x 100 ml.). The combined ethyl acetate extracts were washed with brine (10 ml.), dried over magnesium sulfate and rotary evaporated to give 4.05 g. of the title compound. Recrystallization from acetonitrile (5 ml.) provided 3.1 g. of the title compound m.p. 105 - 107°C.

b) trans-3,3-Diethoxy-2-(hydroxymethyl)cyclobutane carboxylic acid

To a solution of trans-3,3-diethoxy-1,2-cyclobutanedicarboxylic acid, 2-ethyl ester (12.5 g., 48 mmole) in dry tetrahydrofuran (97 ml.) at 0°C. was added lithium triethylborohydride (151 ml., 151 mmole, 1M in tetrahydrofuran) via a dropping funnel. The solution was warmed to room temperature. After one hour at room temperature, the reaction was quenched by the addition of methanol (20 ml.). The solvent was removed by rotary evaporation and the residue was azeotroped with methanol (3 x 300 ml.) to remove remaining borates. The resulting clear gum was dissolved in water and continuous extraction with ethyl acetate for 6.5 hours yielded 7.14 g. of an orange solid. Further continuous extraction with ethyl acetate overnight gave another 1.5 g. of crude product. Total yield of the reaction was 8.64 g. of the title compound.

c) trans-3,3-Diethoxy-2-(hydroxymethyl)cyclobutanecarboxylic acid, methyl ester

To a solution of trans-3,3-diethoxy-2-(hydroxymethyl) cyclobutanecarboxylic acid (8.64 g., 39.6 mmole) in dry dimethylformamide (80 ml.) at room temperature were added methyl iodide (10.0 g., 119 mmole) and sodium bicarbonate (7.4 g., 118 mmole). The mixture was heated at 40°C for 20.5 hours. Rotary evaporation of the solvent gave a solid residue. This residue was taken up in ethyl acetate, stirred for one hour, and filtered. The solids were rinsed repeatedly with ethyl acetate, and rotary evaporation of the combined filtrates gave 11 g. of an orange solid. Flash chromatography on silica (20 - 40% ethyl acetate-hexane) afforded 7.13 g. of the title compound.

d) trans-3,3-Diethoxy-2-[(phenylmethoxy)methyl]cyclobutanecarboxylic acid, methyl ester

To a solution of trans-3,3-diethoxy-2-(hydroxymethyl)cyclobutanecarboxylic acid, methyl ester (3.0 g., 12.9 mmole) in dry cyclohexane (50 ml.), dry methylene chloride (25 ml.), and dry tetrahydrofuran (3.2 ml.)

were added benzyl trichloroacetimidate (4.8 ml., 26 mmole) and trifluoromethanesulfonic acid (100 µl.). The mixture was stirred under argon for 15 minutes when formation of a white precipitate was observed. After one hour, pyridine (0.5 ml.) and hexane (200 ml.) were added to the reaction mixture. The mixture was filtered and then washed with saturated sodium bicarbonate (3 x 100 ml.). The organic layer was dried over sodium sulfate, and rotary evaporation of the solvent afforded 7.4 g. of crude material. Flash chromatography (5 - 10% ethyl acetate-hexane) provided 2.77 g. (about 90% pure by NMR) of the title compound.

e) cis-3,3-Diethoxy-1-fluoro-2-[(phenylmethoxy)methyl]cyclobutanecarboxylic acid, methyl ester

To a solution of dry diisopropylamine (0.76 ml., 4.34 mmole) in dry tetrahydrofuran (24 ml.) at -78°C. was added n-butyllithium (1.8 ml., 3.88 mmole, 2.15 M in hexane). The solution was warmed briefly to -20°C., and then cooled to -78°C. After 30 minutes, a solution of trans-3,3-diethoxy-2-[(phenylmethoxy)methyl]cyclobutanecarboxylic acid, methyl ester (1.0 g., 3.10 mmole) in dry tetrahydrofuran (4 ml.) was added over 6 minutes. Residual ester was transferred by rinsing with dry tetrahydrofuran (2 ml.). The resulting yellowish brown solution was stirred at -78°C. for 45 minutes. The solution was then warmed to -35°C. over a few minutes, and perchloryl fluoride (2 - 4 g.) was cautiously introduced through a pipette over 30 seconds. The temperature during the addition rose to -10°C. The mixture was purged at -35°C. with argon for 15 minutes and at 0°C. for 10 minutes. The reaction was quenched at 0°C. by the dropwise addition of 1M aqueous potassium iodide (20 ml.). The resulting mixture was warmed to room temperature and stirred for 15 minutes. Ether (200 ml.) was added and the aqueous layer was separated. The organic layer was washed with 5% sodium thiosulfate (3 x 20 ml.) and brine (20 ml.). The organic layer was dried over sodium sulfate and rotary evaporation gave 1.04 g. of an oil. Flash chromatography (5 - 20% ethyl acetate-hexane) gave 247 mg. of trans-3,3-diethoxy-1-fluoro-2-[(phenylmethoxy)methyl]cyclobutanecarboxylic acid, methyl ester ($R_f$ = 0.4; 10% ethyl acetate-hexane, two developments) and 262 mg. of cis-3,3-diethoxy-1-fluoro-2-[(phenylmethoxy)methyl]cyclobutanecarboxylic acid, methyl ester ($R_f$ = 0.3, 10% ethyl acetate-hexane, two developments).

f) cis-3,3-Diethoxy-1-fluoro-2-[(phenylmethoxy)methyl]cyclobutanemethanol

To a solution of diisobutylaluminum hydride (22.2 ml., 22.2 mmole, 1M in methylene chloride) in dry methylene chloride (12 ml.) at -78°C. under argon was added a solution of cis-3,3-diethoxy-1-fluoro-2-[(phenylmethoxy)methyl]cyclobutanecarboxylic acid, methyl ester (3.01 g., 8.85 mmole) in dry methylene chloride (20 ml.) over 20 minutes. Residual ester was rinsed with methylene chloride (16 ml.). The solution was stirred at -78°C. for 30 minutes, and then warmed to 0°C. for 30 minutes. After one hour at 0°C., the solution was warmed to room temperature and quenched with methanol (3.7 ml.) and water (5.0 ml.). The mixture was stirred for 30 minutes, filtered through a Celite plug, and the Celite was washed repeatedly with ethyl acetate. Rotary evaporation afforded 2.55 g. of the title compound.

g) cis-1,1-Diethoxy-3-fluoro-2,3-bis[(phenylmethoxy)methyl]cyclobutane

To a suspension of 60% sodium hydride (530 mg., 13.2 mmole) in dry dimethylformamide (10 ml.) was added benzyl bromide (1.57 ml., 13.2 mmole) in one portion at room temperature. Then, cis-3,3-diethoxy-1-fluoro-2-[(phenylmethoxy)methyl]cyclobutanemethanol (2.74 g., 8.78 mmole) in dry dimethylformamide (10 ml.) was added dropwise. Residual alcohol was rinsed with dry dimethylformamide (15 ml.). The solution was stirred at room temperature for three hours. The reaction mixture was quenched with methanol (1 ml.) and stirred for 5 minutes. The reaction mixture was then diluted with diethyl ether (100 ml.), and washed with water (2 x 50 ml.), saturated sodium bicarbonate (2 x 50 ml.), and saturated sodium chloride (2 x 50 ml.). The organic layer was dried over sodium sulfate, filtered and rotary evaporated to yield 3.54 g. of a yellow oil. Flash chromatography on silica (0-5% ethyl acetate-hexane) afforded 2.02 g. of the title compound as a clear oil.

h) cis-3-Fluoro-2,3-bis[(phenylmethoxy)methyl]cyclobutanone

A solution of cis-1,1-diethoxy-3-fluoro-2,3-bis[(phenylmethoxy)methyl]cyclobutane (1.85 g., 7.8 mmole) in acetonitrile (72 ml.) at room temperature was treated with 0.5 M sulfuric acid (36 ml.) and placed in a 60°C. bath for 1.5 hours. The solution was cooled to room temperature, and diluted with ethyl acetate (60 ml.). The mixture was washed with water (2 x 60 ml.), saturated aqueous sodium bicarbonate (2 x 60 ml.), and brine (2 x 60 ml.). The organic layer was dried over sodium sulfate, filtered, and the solvent was removed by rotary evaporation to give 1.5 g. of the title compound as an oil. The oil was rotary evaporated (3 x 30 ml.) in the presence of toluene to remove moisture.

i) (1α,2α,3α)-3-Fluoro-2,3-bis[(phenylmethoxy)methyl]cyclobutanol

To a solution of lithium trisiamylborohydride (2.2 ml., 2.2 mmole) in dry tetrahydrofuran (10 ml.) at -78°C. was added, via cannula, a cold (-78°C.) solution of cis-3-fluoro-2,3-bis[(phenylmethoxy)methyl]cyclobutanone (1.5 g., 7.8 mmole) in dry tetrahydrofuran (20 ml.). The mixture was stirred at -78°C for 20 minutes and then warmed to 0°C. for 15 minutes. The reaction conditions was quenched by the slow addition of saturated aqueous sodium bicarbonate (27 ml.) and 30% hydrogen peroxide (15 ml.). The mixture was stirred for 15 minutes. The mixture was extracted with ethyl acetate (3 x 100 ml.), and the organic layer was dried over sodium sulfate. Rotary evaporation of the solvent provided 1.43 g. of crude title compound. Three successive flash chromatographic separations on silica (5 - 10% ethyl acetate-methylene chloride) yielded 688 mg. of the title compound.

j) (1α,2α,3α)-3-Fluoro-2,3-bis[(phenylmethoxy)methyl]cyclobutanol, 4-methylbenzenesulfonate

To a solution of (1α,2α,3α)-3-fluoro-2,3-bis[(phenylmethoxy)methyl]cyclobutanol (648 mg., 1.96 mmole) in dry pyridine (3.2 ml.) at room temperature was added p-toluenesulfonyl chloride (1.89 g., 9.91 mmole). The mixture was heated at 60°C. for 7 hours. The reaction mixture was cooled to room temperature and diluted with diethyl ether (50 ml.). The ether layer was washed with water (2 x 20 ml.), saturated sodium bicarbonate (2 x 20 ml.), and saturated sodium chloride (1 x 20 ml.). The organic layer was dried over sodium sulfate, filtered and rotary evaporated to produce 2.73 g. of a tan solid. Flash chromatography on silica gel afforded 862 mg. of the title compound.

k) (1α,2β,3β)-9-[3-fluoro-2,3-bis[(phenylmethoxy)methyl]cyclobutyl]-6-(phenylmethoxy)-9H-purin-2-amine

To a solution of (1α,2α,3α)-3-fluoro-2,3-bis[(phenylmethoxy)methyl]cyclobutanol, 4-methylbenzenesulfonate (820 mg., 1.69 mmole) in dry dimethylformamide (1.1 ml.) were added 2-amino-6-(phenylmethoxy)-9H-purine (1.3 g., 5.39 mmole), potassium carbonate (1.0 g., 7.24 mmole), and 18-crown-6 (1.48 g., 5.60 mmole). The mixture was heated at 110°C. for 14 hours. The reaction mixture was then cooled to room temperature, diluted with ethyl acetate, and filtered through a Celite plug. The Celite was rinsed repeatedly with ethyl acetate and then with methylene chloride. Rotary evaporation of the solvent gave 4.06 g. of a dark brown oil. Flash chromatography on silica (25% - 33% ethyl acetate - hexane) afforded 180 mg. of impure product. This material was rechromatographed on silica (50% - 90% diether ether - hexane) to yield 124 mg. of the pure title compound.

l) (1α,2β,3β)-2-Amino-9-[3-fluoro-2,3-bis(hydroxymethyl)cyclobutyl]-1,9-dihydro-6H-purin-6-one

To a solution of (1α,2β,3β)-9-[3-fluoro-2,3-bis[(phenylmethoxy)methyl]cyclobutyl]-6-(phenylmethoxy)-9H-purin-2-amine (96 mg., 0.17 mmole) in absolute ethanol (8 ml.) were added cyclohexene (4 ml.) and palladium hydroxide on carbon (20%, 50 mg.). The mixture was heated at reflux for 14 hours and the hot mixture was filtered through Celite. The Celite was then rinsed with hot methanol and hot water, until no additional mass was recovered upon rotary evaporation, to give 50 mg. of a white solid. This solid was suspended in water and chromatographed (CHP-20 resin; 0 - 40% acetonitrile - water) to afford 44 mg. of material. This material was then suspended in water and freeze-dried to provide 41 mg. of the title compound as a white solid; m.p. 220° (dec.). $^1H$ NMR (dimethylsulfoxide -$d_6$) δ 2.45 - 2.60 (m, 1H), 2.60 - 2.80 (m, 1H), 3.05 - 3.20 (m, 1H), 3.50 - 3.80 (m, 4H), 4.60 (br s, 1H), 4.66 (dd,1H, J=9.0 Hz), 5.11 (brs, 1H), 6.43 (br s, 2H), 7.82 (S, 1H).

Example 2

(1α,2β,3β)-2-Amino-9-[3-fluoro-2-hydroxy-3-(hydroxymethyl)cyclobutyl]-1,9-dihydro-6H-purin-6-one

a) Diethyl fluoromalonate

This compound was prepared by the method of Ishikawa et al. [see J. Fluorine Chem., 25, 203 (1984)].

Hexafluoropropane gas (38.0 g., 0.25 mole) was introduced via a gas equilibrium assembly (Ace Glass) into a 0°C. solution of sodium ethoxide in dry ethanol (2.9 M, 60 ml.). The gas was added over 2.5 hours. After an additional one hour of stirring at room temperature, the reaction mixture was poured into distilled water (300 ml.) and a clear oil resulted. The oily layer (58.8 g.) was separated, placed in a polyethylene bottle, cooled to 0°C., and concentrated sulfuric acid (40 ml.) was added dropwise while keeping the temperature below about

30°C. The mixture was then stirred for one hour at room temperature, poured into ice water, and the resulting oily layer was separated. The oil was washed with saturated aqueous sodium bicarbonate and then with water. The residue was dried over magnesium sulfate, and distillation gave 23 g. of ethyl 2,3,3,3-tetrahydrofluoropropionate; b.p. 95 - 98°C.

To 3.3 M sodium ethoxide (100 ml.) was added ethyl 2,3,3,3-tetrafluoropropionate in a dropwise manner, keeping the temperature below 30°C. After 30 minutes of stirring at room temperature, the mixture was acidified with concentrated HCl (15.5 ml.). The resulting suspension was stirred for one hour at room temperature, and then filtered.

The filtrate was poured into ice water. The oily material was extracted with ether, and the ethereal extracts were washed with saturated aqueous sodium bicarbonate, and then with brine. The organic layer was dried over magnesium sulfate. Distillation under pressure gave 17.1 g. of diethyl fluoromalonate; b.p. 55 - 56°C/4 mm Hg.

b) Fluoro[(phenylmethoxy)methyl]propanedioic acid, diethyl ester

In a 500 ml. flask, sodium hydride (4.08 g., 102 mmole, 60% dispersion in mineral oil) was suspended in dry tetrahydrofuran (100 ml.). The suspension was cooled to -20°C., and benzyl chloromethyl ether (18.6 ml., 134 mmole) was added via syringe. To the resulting mixture was added a solution of diethyl fluoromalonate (12 g., 67.4 mmole) in dry tetrahydrofuran (300 ml.) via addition funnel (rinsed with 5 ml. of solvent). The mixture was warmed to 0°C. and stirred for 3 hours. Then, the mixture was warmed to room temperature and stirred for 1.5 hours. The mixture was cooled to 0°C. and quenched with dry ethanol (20 ml.). The reaction solution was stirred at room temperature for 30 minutes, and then slowly poured into saturated ammonium chloride (300 ml.) at 0°C. The mixture was diluted with diethyl ether (300 ml.) and separated. The aqueous layer was further extracted with diethyl ether (2 x 100 ml.), and the combined organic layers were washed with saturated aqueous sodium bicarbonate (2 x 100 ml.). The organic layer was dried over sodium sulfate, filtered, and the solvent was removed by rotary evaporation to afford 31 g. of a brown oil. Flash chromatography (16 - 20% ethyl acetate-hexane) afforded 18.8 g. of the title compound.

c) 2-Fluoro-2-[(phenylmethoxy)methyl]-1,3-propanediol

To a suspension of lithium borohydride (3.6 g., 165 mmole) in anhydrous diethyl ether (50 ml.) at 0°C. was added over one hour a solution of fluoro[(phenylmethoxy)methyl]propanedioic acid, diethyl ester (18.8 g., 63.0 mmole) in anhydrous diethyl other (86 ml.). The mixture was warmed to room temperature and stirred for 12.5 hours. The reaction mixture was cooled to 0°C. and quenched by the addition of methanol (70 ml.). The mixture was then warmed to room temperature and treated with AGMP resin (74 g.) to achieve a pH of 4. The mixture was stirred at room temperature for 30 minutes, filtered, and the solvent was removed by rotary evaporation. The residue was dissolved in methanol (80 ml.) and evaporated. This process was repeated twice more to give 13.1 g. of tan solid. Flash chromatography (5% methanol-methylene chloride) afforded 12.4 g. of the title compound as a white solid; m.p. 77-79°C.

d) 2-Fluoro-2-[(phenylmethoxy)methyl]-1,3-propanediol, di(trifluoromethanesulfonate)

A solution of 2-fluoro-2-[(phenylmethoxy)methyl]-1,3-propanediol (12.3 g., 57.4 mmole) dissolved in methylene chloride (216 ml.) and pyridine (24 ml.) was added over 30 minutes to a solution of trifluoromethanesulfonic anhydride (40.1 g., 142 mmole) in dry methylene chloride (24 ml.) at -80°C. The resulting mixture was warmed to 0°C. for 30 minutes. The reaction mixture was then diluted with methylene chloride (100 ml.), and washed with water (3 x 50 ml.) and saturated sodium bicarbonate (3 x 50 ml.). The organic layer was dried over sodium sulfate, filtered, and rotary evaporated to give 28.0 g. of the title compound as a green oil.

e) cis- and trans-1-Fluoro-3-(methylsulfinyl)-3-methylthio-1-[(phenylmethoxy)methyl]cyclobutane

To a solution of methyl methylsulfinylmethyl sulfide (21.4 g., 172 mmole) in dry tetrahydrofuran (90 ml.) at 0°C. was added via syringe 2.22 M n-butyllithium (64 ml., 143 mmole) in hexane. The resulting solution was stirred for 15 minutes at 0°C., and then cooled to -78°C. A solution of 2-fluoro-2-[(phenylmethoxy)methyl]-1,3-propanediol, di(trifluoromethanesulfonate) (27.5 g., 57.4 mmole) dissolved in tetrahydrofuran (100 ml.) was added over 30 minutes resulting in a dark orange-red color. The reaction was then kept at -80°C. overnight. The reaction was diluted with methylene chloride (100 ml.), and washed with water (2 x 100 ml.), and then saturated aqueous sodium bicarbonate (2 x 100 ml.). The organic layer was dried over sodium sulfate and rotary

evaporation gave 20.6 g. of a dark brown oil. Flash chromatography (20 - 25% ethyl acetate-hexane) afforded 7.36 g. of the title compound as a mixture of diastereomers.

f) 1-Fluoro-3,3-dimethoxy-1-[(phenylmethoxy)methyl]cyclobutane

To a solution of cis- and trans-1-fluoro-3-(methylsulfinyl)-3-methylthio-1-[(phenylmethoxy)methyl]cyclobutane (7.36 g., 24.3 mmole) in dry methanol (225 ml.) at 0°C. were added trimethylorthoformate (30 ml.) and concentrated sulfuric acid (0.5 ml.). The solution was heated to 80°C. for four hours, cooled to room temperature, and diluted with methylene chloride. The resulting mixture was washed with water (2 x 100 ml.), saturated aqueous sodium bicarbonate (2 x 100 ml.), and brine (2 x 100 ml.). The organic layer was dried over sodium sulfate, filtered, and rotary evaporated to give 6.6 g. of a dark brown oil. This material was combined with another batch (22 mmole) of crude dimethyl ketal and flash chromatography (2:3 hexane-ethyl acetate) afforded 9.7 g. of the title compound.

g) 3-Fluoro-3-[(phenylmethoxy)methyl]cyclobutanone

A solution of 1-fluoro-3,3-dimethoxy-1-[(phenymethoxy)methyl]cyclobutane (2.0 g., 7.8 mmole) in acetonitrile (15 ml.) at room temperature was warmed to 60°C. and treated with 0.5 M sulfuric acid (5.3 ml.). After 40 minutes at 60°C., the solution was cooled to room temperature, and diluted with ethyl acetate (100 ml.). The mixture was washed with water (100 ml.), saturated aqueous sodium bicarbonate (1 x 100 ml.), and brine (1 x 100 ml.). The organic layer was dried over sodium sulfate, filtered, and the solvent removed by rotary evaporation to give 1.63 g. of the title compound as an orange oil.

h) cis- and trans-3-Fluoro-3-[(phenylmethoxy)methyl]cyclobutanol

To a solution of lithium trisiamylborohydride (2.2 ml., 2.2 mmole) in dry tetrahydrofuran (13 ml.) at -78°C. was added, via cannula, a -78°C. solution of 3-fluoro-3-[(phenylmethoxy)methyl]cyclobutanone (1.63 g., 7.8 mmole) in dry tetrahydrofuran (13 ml.). The mixture was stirred at -78°C. for 20 minutes and at 0°C. for 15 minutes. The reaction was quenched at 0°C. by the slow addition of saturated aqueous sodium bicarbonate (27 ml.) and 30% hydrogen peroxide (15 ml.). The mixture was stirred for 15 minutes and then extracted with ethyl acetate (3 x 100 ml.). The organic layer was dried over sodium sulfate and the solvent was removed by rotary evaporation to give 1.63 g. of the title compound as a 4:1 (cis:trans) mixture of diastereomers.

i) cis- and trans-3-Fluoro-3-[(phenylmethoxy)methyl]cyclobutanol, 4-methylbenzenesulfonate

To a solution of cis- and trans-3-fluoro-3-[(phenylmethoxy)methyl]cyclobutanol (1.63 g., 7.61 mmole) in pyridine (8 ml.) was added p-toluenesulfonyl chloride (5.53 g., 29.0 mmole). The mixture was heated to 60°C. for one hour. The reaction was cooled to room temperature, diluted with diethyl ether, and washed with water (2 x 100 ml.), saturated aqueous sodium bicarbonate (1 x 100 ml.), and brine (1 x 100 ml.). The organic layer was dried over sodium sulfate, and rotary evaporation of the solvent gave 3.0 g. of a solid. Flash chromatography (12% - 16% ethyl acetate-hexane) afforded 1.68 g. of the title compound.

j) 3-Fluoro-3-[(phenylmethoxy)methyl]cyclobutene

To a solution of potassium tert-butoxide (539 mg., 4.8 mmole) in dimethylsulfoxide (4 ml.) was added a solution of cis- and trans-3-fluoro-3-[(phenylmethoxy)methyl]cyclobutanol, 4-methylbenzenesulfonate (897 mg., 2.46 mmole) in dimethylsulfoxide (3 ml.) with stirring under argon. After one hour, the red solution was poured into water and diluted with methylene chloride. The aqueous layer was extracted twice with methylene chloride and the combined organics were washed with brine and dried over sodium sulfate. After removing the volatiles in vacuo, the title compound (525 mg.), contaminated with about 25 mole % of dimethylsulfoxide, was used directly in the subsequent reaction.

k) (1α,2β,4α)-and (1α,2α,4α)-2-Fluoro-2-[(phenylmethoxy)methyl]-5-oxabicyclo[2.1.0]pentane

A mixture of 3-fluoro-3-[(phenylmethoxy)methy]cyclobutene (525 mg., about 2.4 mmole, crude product from previous reaction), 3-chloroperoxybenzoic acid (850 mg., 75% pure, 3.69 mmole), and 3-t-butyl-4-hydroxy-5-methylphenyl sulfide (7 mg.) in 1,2-dichloroethane (8.2 ml.) was stirred in a sealed tube at 53°C. for 69 hours. The reaction was added to a mixture of methylene chloride, saturated sodium carbonate and sodium

thiosulfate at 18°C. After stirring at room temperature for 30 minutes, the aqueous layer was extracted twice with methylene chloride. The combined organic layers were dried over sodium sulfate and concentrated to an oily-solid. The residue was purified by flash chromatograph (CC-7 silica gel, 230-400 mesh), eluting with methylene chloride:hexanes (3:2) to give 115 mg. of (1α,2α,4α)-2-fluoro-2-[(phenylmethoxy)methyl]-5-oxabicyclo[2.1.0]pentane and another 84 mg. of a mixed fraction containing about 80% of (1α,2α,4α)-2-fluoro-2-[(phenylmethoxy)methyl]-5-oxabicyclo[2.1.0]pentane and 20 % of (1α,2β,4α)-2-fluoro-2-[(phenylmethoxy)methyl]-5-oxabicyclo [2.1.0]pentane.

l) (1α,2α,4β)-4-[2-Amino-6-(phenylmethoxy)-9H-purin-9-yl]-2-fluoro-2-[(phenylmethoxy)methyl] cyclobutanol

Lithium hydride (4.7 mg., 0.59 mmole) was added to a solution of (1α,2α,4α)-2-fluoro-2-[(phenylmethoxy)methyl]-5-oxabicyclo[2.1.0]pentane (204 mg., 0.982 mmole) and 2-amino-6-(phenylmethoxy)-9H-purine (610 mg., 2.54 mmole) in dimethylformamide (6.6 ml.). The reaction was stirred at 110°C. for 34 hours, then stirred for an additional 12 hours at room temperature. Concentration under vacuum at 45°C gave an orange, oily-foam. The residue was purified by flash chromatography on silica gel eluting with methanol-methylene chloride (0.5, 1 and finally 2% methanol) to give 239 mg. of the title compound as an oily foam.

m) (1α,2β,3β)-2-Amino-9-(3-fluoro-2-hydroxy-3-(hydroxymethyl)cyclobutyl]-1,9-dihydro-6H-purin-6-one

A mixture of (1α,2α,4β)-4-[2-amino-6-(phenylmethoxy)-9H-purin-9-yl]-2-fluoro-2-[(phenylmethoxy)methyl]cyclobutanol (239 mg., 0.532 mmole), cyclohexene (8 ml.), and 20% palladium hydroxide on carbon (109 mg.) in 95% ethanol (16 ml.) was refluxed under argon for 19.5 hours. The still hot reaction mixture was filtered through a Celite pad, and the filter cake was washed thoroughly with hot water and hot methanol. The combined filtrates were evaporated in vacuo. The residue was slurried in water and purified on a CHP-20P resin column, eluting first with water, then acetonitrile-water (5, 7, 10, 15, 20, and finally 25% acetonitrile). The fractions containing pure compound were concentrated in vacuo, and the residue (135 mg.) was slurried in water and lyophilized to give 134 mg. of the title compound as a colorless solid; m.p. 200 - 210°C. (dec.). $^1$H NMR (dimethylsulfoxide - $d_6$) δ10.68 (br s, 1H); 7.92 (S, 1H); 6.5 (br s, 2H), 5.97 (d, J = 7.3 Hz, 1H); 5.12 (t, J = 5.6 Hz, 1H); 4.76 - 4.83 (m, 1H); 4.53 - 4.61 (m, 1H); 3.62 - 3.78 (m, 2H); 2.26 - 2.47 (m, 2H).

Example 3

Treatment of Viral Infection in Cell Culture in Vitro

Assays were performed in cell culture systems to determine the concentrations of compounds that are effective in preventing several kinds of viral infections. The assays are described below, and the results are presented in the Table.

Abbreviations:

HSV-1 (herpes simplex virus type 1, strain Schooler), HSV-2 (herpes simplex virus type 2, strain 186), VZV (varicella zoster virus, strain ELLEN), HCMV (human cytomegalovirus, strain AD 169).

HSV-1, HSV-2, HCMV, and VZV antiviral assays: Virus was adsorbed to WI-38 cell culture monolayers in 6 cell culture plates (Costar, Cambridge, MA) for 1 hour prior to addition of maintenance medium containing duplicate dilutions of the test compound. Inhibition of plaque development was evaluated on fixed and stained monolayers after 4 days incubation at 37°C for HSV-1 and HSV-2 and after 6-7 days incubation at 37°C for HCMV and VZV. $ID_{50}$ values were determined from the drug concentration which conferred at least a 50% plaque reduction compared to virus controls.

TABLE

| ID$_{50}$ (μM) for the following viruses | | | | |
|---|---|---|---|---|
| Compound | HSV-1 | HSV-2 | VZV | HCMV |
| Product of Example 1 | 0.35-0.70 | 0.35-0.70 | 1.1 | 7-18 |
| Product of Example 2 | 37 - 93 | 37 - 93 | 7-19 | 37 |

## Claims

1. A compound having the formula

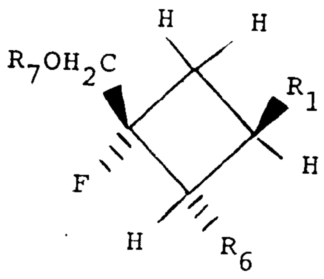

or such a compound in pharmaceutically acceptable salt form wherein:
$R_1$ is

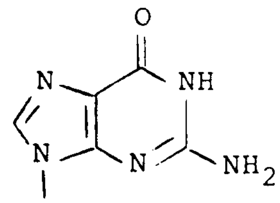 , 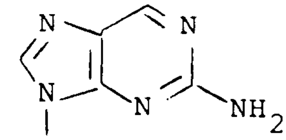 ,

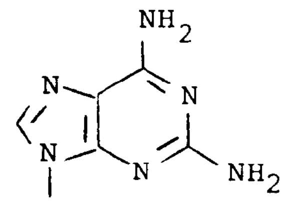 , 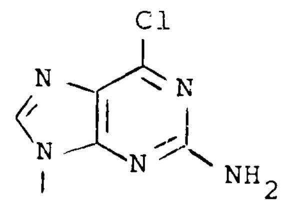 ,

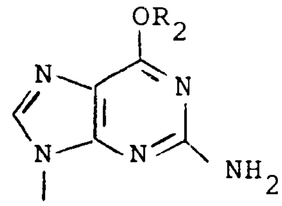 , 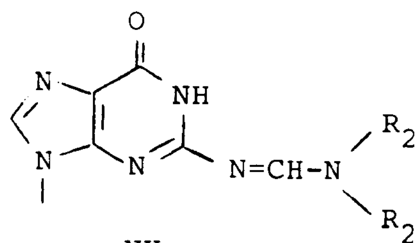 ,

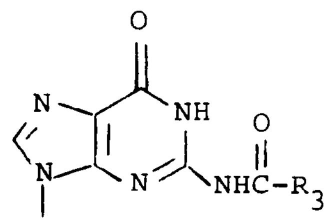 , 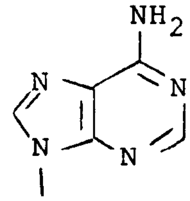 ,

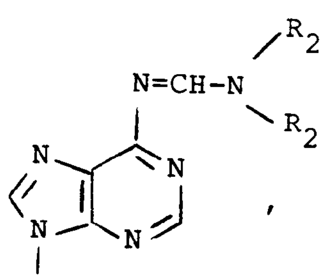 , 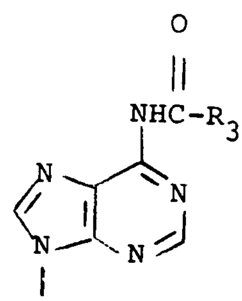 ,

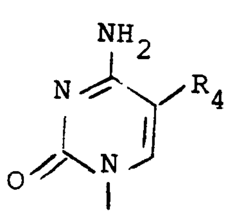 , or 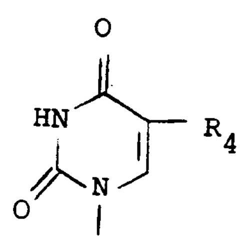 ;

$R_2$ is straight or branched chain alkyl of 1 to 10 carbons;

$R_3$ is hydrogen, aryl, straight or branched chain alkyl of 1 to 10 carbons, straight or branched chain alkyl having one or more substituents selected from the group consisting of bromo, chloro, fluoro, iodo,

amino, hydroxy, cyano, trialkylamino wherein each alkyl is of 1 to 6 carbons, alkoxy of 1 to 6 carbons, aryl, and carboxy;

$R_4$ is fluoro, chloro, bromo, iodo, hydrogen, methyl, trifluoromethyl, ethyl, n-propyl, 2-fluoroethyl, 2-chloroethyl, or

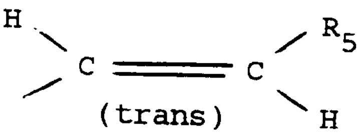

wherein $R_5$ is chloro, bromo, iodo, hydrogen, methyl or trifluoromethyl;

$R_6$ is -OH, $-CH_2OH$, $-OPO_3H_2$, $-CH_2-OPO_3H_2$

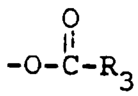 or 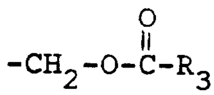 ;

and

$R_7$ is hydrogen, $-PO_3H_2$, or

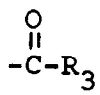 ;

the term "aryl" refers to phenyl and phenyl having one, two, or three substituents selected from the group consisting of alkyl of 1 to 6 carbons, alkoxy of 1 to 6 carbons, bromo, chloro, fluoro, iodo, trifluoromethyl, amino, alkylamino of 1 to 6 carbons, dialkylamino wherein each alkyl is of 1 to 6 carbons, nitro, cyano, alkanoyloxy of 2 to 11 carbons, carboxy, carbamoyl, and hydroxy.

**2.** A compound of Claim 1 wherein
$R_1$ is

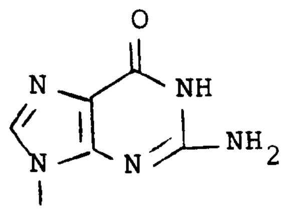 , 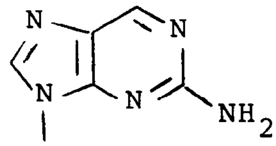 ,

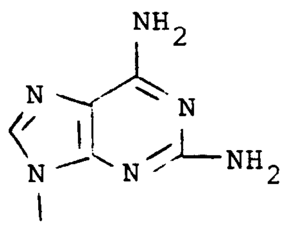 , 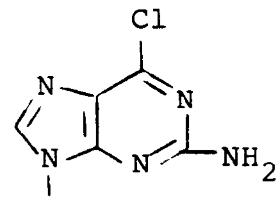 ,

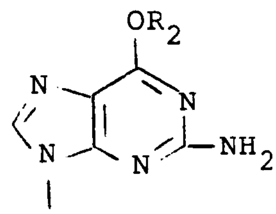 , 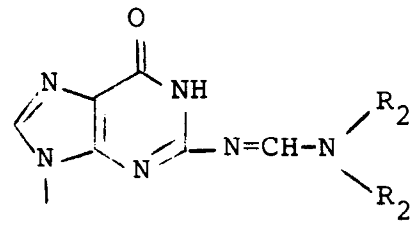 ,

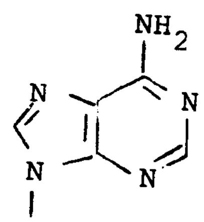 , 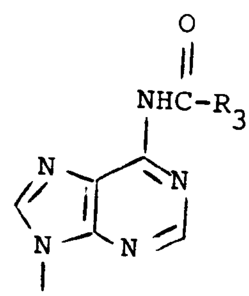 ,

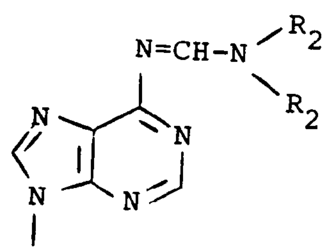 , or 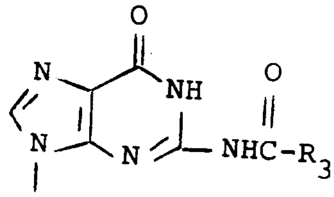 .

3. A compound of Claim 1 wherein $R_6$ is -OH or -$CH_2$ OH and $R_7$ is hydrogen.

4. A compound of Claim 3 wherein $R_1$ is

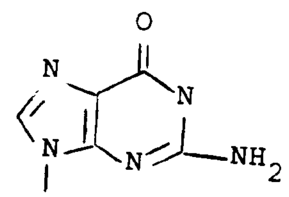

.

5. A compound of Claim 4 wherein $R_6$ is $-CH_2OH$.

6. A compound of Claim 4 wherein $R_6$ is -OH.

7. A compound of the formula

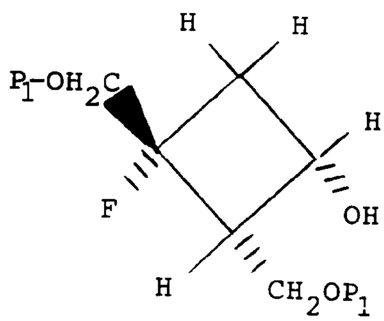

or of the formula

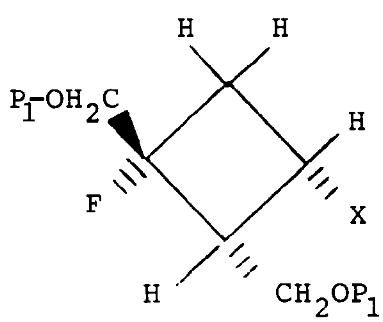

wherein $P_1$ is a hydroxy protecting group and X is a leaving group.

8. The compound of the formula

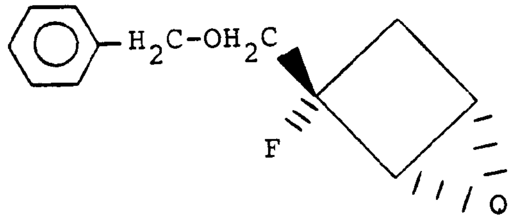

.

**9.** The compound of the formula

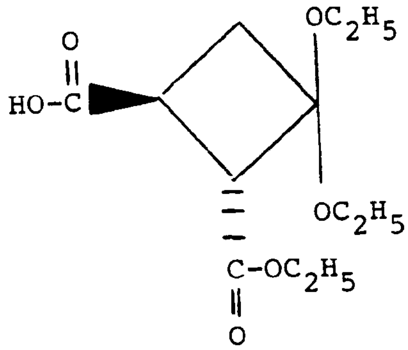

.

**10.** A process for preparing the compound of Claim 9 which comprises reacting a compound of the formula

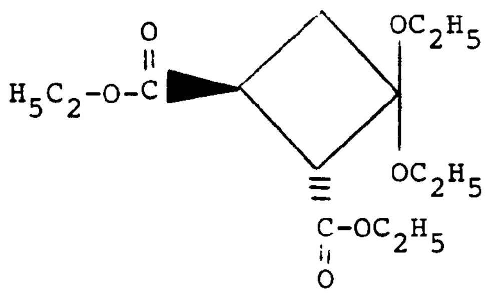

with an aqueous solution of lithium hydroxide in a solvent.